# EUROPEAN PATENT APPLICATION

(11) **EP 4 249 487 A2**
(43) Date of publication of application: **27.09.2023**
(21) Application number: 23166821.1
(22) Date of filing: 16.10.2020
(51) Int. Cl.: C07D 405/04

(54) **METHODS FOR THE PREPARATION OF 5-BROMO-2-(3-CHLORO-PYRIDIN-2-YL)-2H-PYRAZOLE-3-CARBOXYLIC ACID**

(30) Priority: 18.10.2019 US 201962916832 P; 06.11.2019 US 201962931310 P
(62) Divisional of application: 20804378.6
(71) Applicant: FMC Corporation, Philadelphia, Pennsylvania 19104 (US); FMC Agro Singapore Pte. Ltd., Singapore 018983 (SG)
(72) Inventor: CAO, Yanchun, Philadelphia, PA 19104 (US); CHEN, Liang, Philadelphia, PA 19104 (US); MAO, Jianhua, Philadelphia, PA 19104 (US); XU, Zhijian, Philadelphia, PA 19104 (US)
(74) Representative: Dehns

(57) **Abstract**

The invention is directed to methods for preparing intermediates which are useful for making insecticidal anthranilamide compounds such as chlorantraniliprole and cyantraniliprole.

## Description

### CROSS-REFERENCE TO RELATED APPLICATION

This application claims the benefit of U.S. Provisional Application No. 62/916,832 filed October 18, 2019 and of U.S. Provisional Application No. 62/931,310 filed November 6, 2019.

### FIELD OF INVENTION

This disclosure is directed to novel methods of synthesizing 5-Bromo-2-(3-chloro-pyridin-2-yl)-2H-pyrazole-3-carboxylic acid. Compounds prepared by the methods disclosed herein are useful for preparation of certain anthranilamide compounds that are of interest as insecticides, such as, for example, the insecticides chlorantraniliprole and cyantraniliprole.

### BACKGROUND

Conventional processes for the production of 5-Bromo-2-(3-chloro-pyridin-2-yl)-2H-pyrazole-3-carboxylic acid are subject to several industrial concerns, such as processability, environmental hazards, high cost, reagent reactivity, and necessary specialized equipment.

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, eliminated need for mixed solvent separations, reduced waste, simplified operation complexity, and reduced process hazards.

The disclosed methods provide an overall yield of about 50% with commercially available and easily handled reagents.

### BRIEF DESCRIPTION

In one aspect, provided herein is a method of preparing a compound of Formula VI, wherein 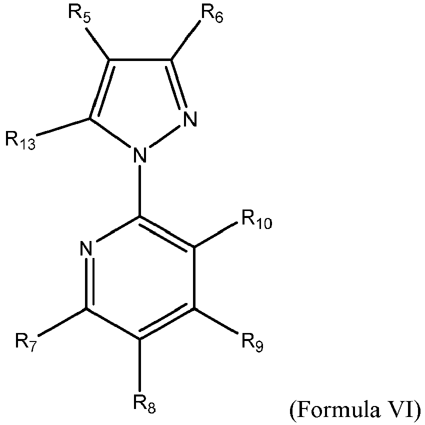
each of R₅ - R₁₀ is independently selected from hydrogen and halogen; and
R₁₃ is an organic acid, the method comprising
   I) forming a mixture comprising
      A) a compound of Formula V, wherein 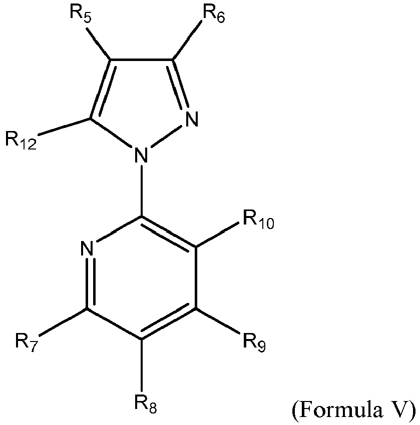
         each of R₅ - R₁₀ is independently selected from hydrogen and halogen;
         R₁₂ is selected from ether, ester, and nitrile; and
         wherein the compound of Formula V is prepared according to a method comprising
            i) forming a mixture comprising
               a) a compound of Formula III, wherein 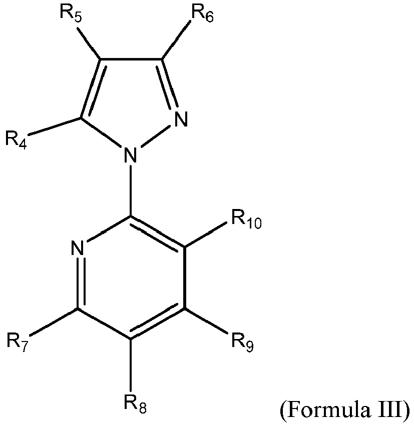
                  R₄ is hydrogen;
                  each of R₅ - R₁₀ is independently selected from hydrogen and halogen; and
                  wherein the compound of Formula III is prepared according to a method comprising
                     IA) forming a mixture comprising
                        AA) a compound of Formula II, wherein 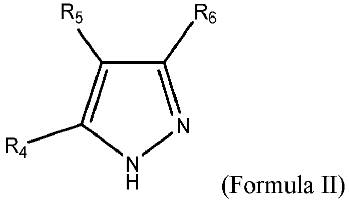
                           each of R₄, R₅, and R₆ is independently selected from hydrogen and halogen;
                           wherein at least one of R₄, R₅, and R₆ is hydrogen; and
                           wherein the compound of Formula II is prepared according to a method comprising
                              ia) forming a mixture comprising
                                 aa) a compound of Formula VIII, wherein 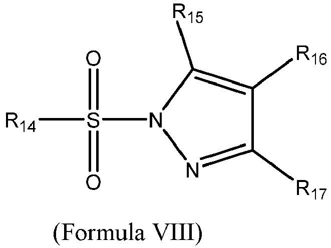
                                    R₁₄ is selected from substituted or unsubstituted aryl, substituted or unsubstituted heteroaryl, substituted or unsubstituted carbocycle, and substituted or unsubstituted heterocycle;
                                    each of R₁₅, R₁₆, and R₁₇ is independently selected from hydrogen and halogen; and
                                    wherein at least one of R₁₅, R₁₆, and R₁₇ is a halogen;
                                 bb) a solvent; and
                                 cc) an inorganic base; and
                              iia) reacting the mixture;
                           BB) a compound of Formula IV, wherein 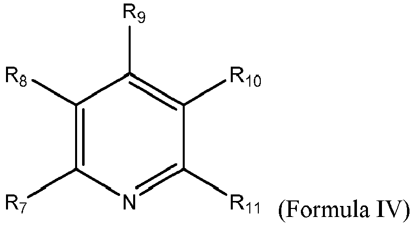 each of R₇ - R₁₁ is independently selected from hydrogen and halogen;
                           CC) a solvent;
                           DD) an inorganic base; and
                           EE) optionally an additive; and
                        IIA) reacting the mixture;
                           b) a solvent;
                           c) an organic compound;
                           d) a compound comprising a metal; and
                           e) optionally an additive; and
                        ii) reacting the mixture; and
                     B) a metal hydroxide; and
                  II) reacting the mixture.

In one aspect, provided herein is a method of preparing a compound of Formula VI, wherein 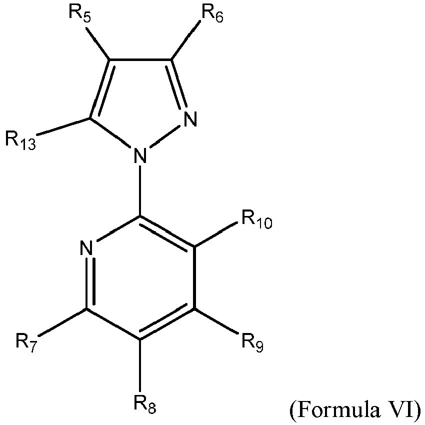
each of R₅ - R₁₀ is independently selected from hydrogen and halogen; and
R₁₃ is an organic acid, the method comprising
   I) forming a mixture comprising
      A) a compound of Formula III, wherein 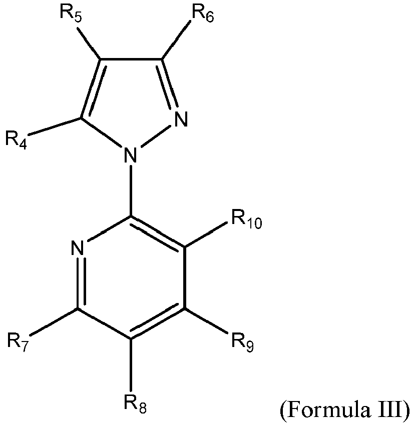
         R₄ is hydrogen;
         each of R₅ - R₁₀ is independently selected from hydrogen and halogen; and
         wherein the compound of Formula III is prepared according to a method comprising
            i) forming a mixture comprising
               a) a compound of Formula II, wherein 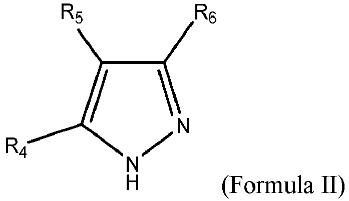
                  each of R₄, R₅, and R₆ is independently selected from hydrogen and halogen;
                  wherein at least one of R₄, R₅, and R₆ is hydrogen; and
                  wherein the compound of Formula II is prepared according to a method comprising
                     IA) forming a mixture comprising
                        AA) a compound of Formula VIII, wherein 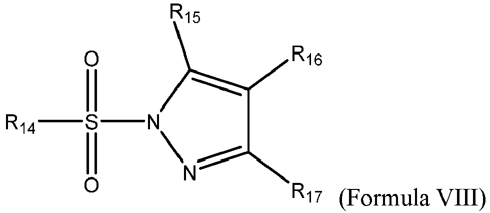
                           R₁₄ is selected from substituted or unsubstituted aryl, substituted or unsubstituted heteroaryl, substituted or unsubstituted carbocycle, and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        BB) a solvent; and
                        CC) an inorganic base; and
                     IIa) reacting the mixture;
               b) a compound of Formula IV, wherein 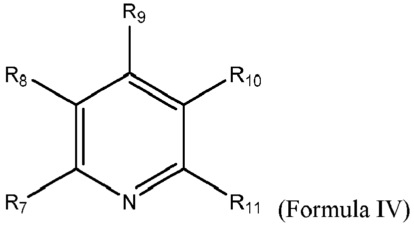 each of R₇ - R₁₁ is independently selected from hydrogen and halogen;
               c) a solvent;
               d) an inorganic base; and
               e) optionally an additive; and
            ii) reacting the mixture;
         B) a carbonyl-containing compound;
         C) a solvent;
         D) a compound comprising a metal; and
         E) optionally an additive; and
   II) reacting the mixture.

In one aspect, provided herein is a method of preparing a compound of Formula II, wherein 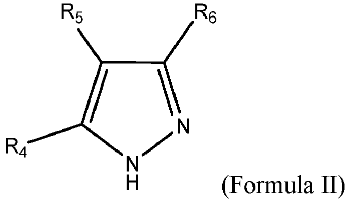
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula VIII, wherein 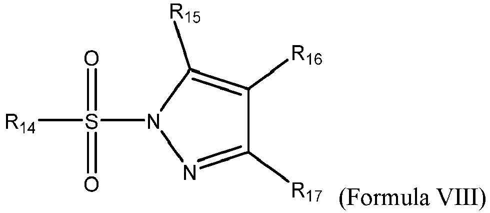
         R₁₄ is selected from substituted or unsubstituted aryl, substituted or unsubstituted heteroaryl, substituted or unsubstituted carbocycle, and substituted or unsubstituted heterocycle;
         each of R₁₅, R₁₆, and R₁₇ is independently selected from hydrogen and halogen; and
         wherein at least one of R₁₅, R₁₆, and R₁₇ is a halogen;
      B) a solvent; and
      C) an inorganic base; and
   II) reacting the mixture.

In one aspect, provided herein is a method of preparing a compound of Formula VII, wherein 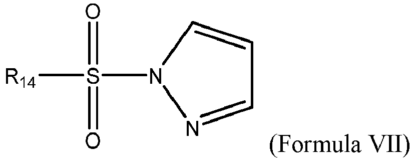 R₁₄ is selected from substituted or unsubstituted aryl, substituted or unsubstituted heteroaryl, substituted or unsubstituted carbocycle, and substituted or unsubstituted heterocycle, the method comprising
I) forming a mixture comprising
   A) pyrazole or a pyrazole derivative;
   B) an organosulfur compound;
   C) optionally a solvent; and
   D) optionally a base; and
II) reacting the mixture.

In one aspect, provided herein is a method of preparing a compound of Formula VIII, wherein 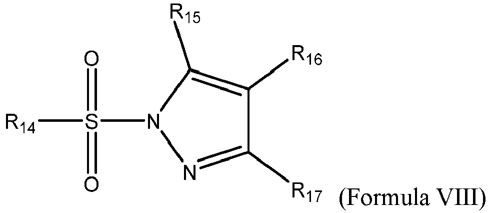
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen, the method comprising
   I) forming a mixture comprising
      A) the compound of Formula VII, wherein 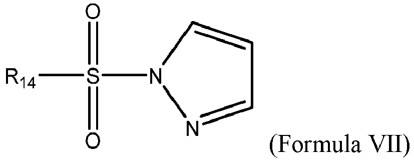 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.

In one aspect, provided herein is a method of preparing a compound of Formula V, wherein 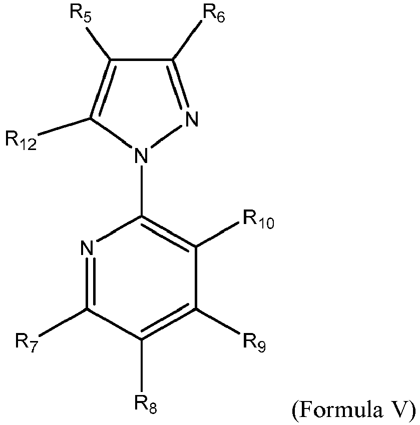
each of R₅ - R₁₀ is independently selected from hydrogen and halogen; and
R₁₂ is selected from ether, ester, and nitrile, the method comprising
   i) forming a mixture comprising
      a) a compound of Formula III, wherein 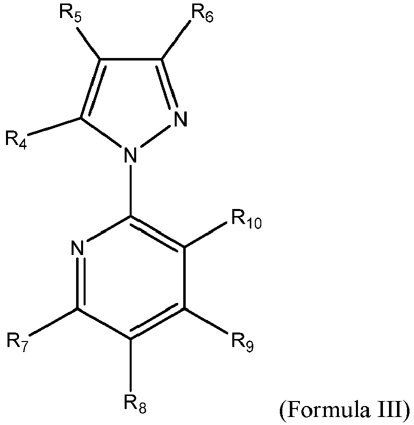
         R₄ is hydrogen;
         each of R₅ - R₁₀ is independently selected from hydrogen and halogen; and
         wherein the compound of Formula III is prepared according to a method comprising
            IA) forming a mixture comprising
               AA) a compound of Formula II, wherein 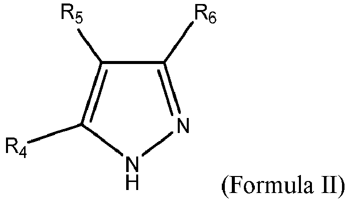
                  each of R₄, R₅, and R₆ is independently selected from hydrogen and halogen;
                  wherein at least one of R₄, R₅, and R₆ is hydrogen; and
                  wherein the compound of Formula II is prepared according to a method comprising
                     ia) forming a mixture comprising
                        aa) a compound of Formula VIII, wherein 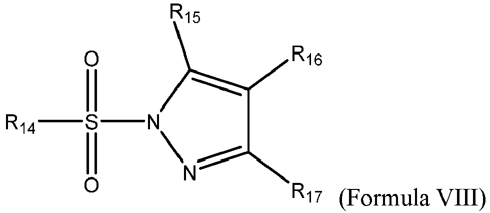
                           R₁₄ is selected from substituted or unsubstituted aryl, substituted or unsubstituted heteroaryl, substituted or unsubstituted carbocycle, and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        bb) a solvent; and
                        cc) an inorganic base; and
                     iia) reacting the mixture;
               BB) a compound of Formula IV, wherein 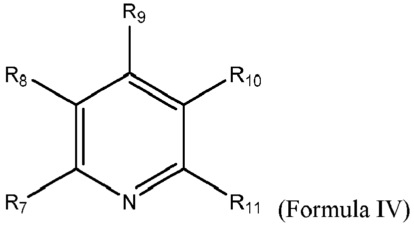 each of R₇ - R₁₁ is independently selected from hydrogen and halogen;
               CC) a solvent;
               DD) an inorganic base; and
               EE) optionally an additive; and
            IIA) reacting the mixture;
               b) a solvent;
               c) an organic compound;
               d) a compound comprising a metal; and
               e) optionally an additive; and
   ii) reacting the mixture.

In one aspect, provided herein is a method of preparing a compound of Formula III, wherein 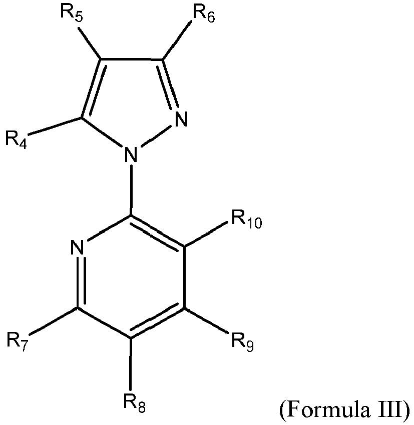
R₄ is hydrogen; and
each of R₅ - R₁₀ is independently selected from hydrogen and halogen, the method comprising
   i) forming a mixture comprising
      a) a compound of Formula II, wherein 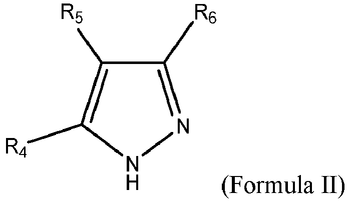
         each of R₄, R₅, and R₆ is independently selected from hydrogen and halogen;
         wherein at least one of R₄, R₅, and R₆ is hydrogen; and
         wherein the compound of Formula II is prepared according to a method comprising
            IA) forming a mixture comprising
               AA) a compound of Formula VIII, wherein 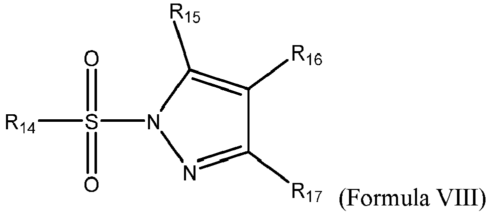
                  R₁₄ is selected from substituted or unsubstituted aryl, substituted or unsubstituted heteroaryl, substituted or unsubstituted carbocycle, and substituted or unsubstituted heterocycle;
                  each of R₁₅, R₁₆, and R₁₇ is independently selected from hydrogen and halogen; and
                  wherein at least one of R₁₅, R₁₆, and R₁₇ is a halogen;
               BB) a solvent; and
               CC) an inorganic base; and
            IIa) reacting the mixture;
      b) a compound of Formula IV, wherein 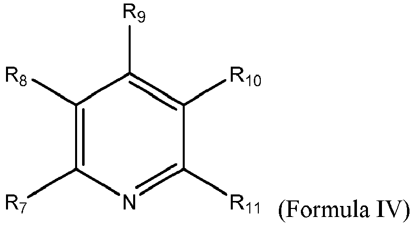 each of R₇ - R₁₁ is independently selected from hydrogen and halogen;
         c) a solvent;
         d) an inorganic base; and
         e) optionally an additive; and
   ii) reacting the mixture.

In one aspect, provided herein is a method of preparing a compound of Formula II-A, wherein 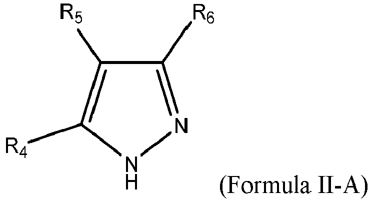
M is selected from alkali metals and alkaline metals;
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula VIII, wherein 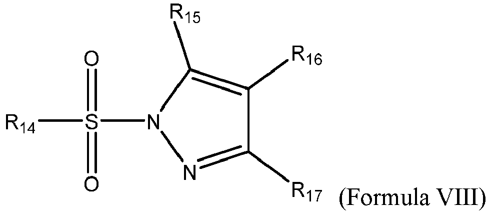
         R₁₄ is selected from substituted or unsubstituted aryl, substituted or unsubstituted heteroaryl, substituted or unsubstituted carbocycle, and substituted or unsubstituted heterocycle;
         each of R₁₅, R₁₆, and R₁₇ is independently selected from hydrogen and halogen; and
         wherein at least one of R₁₅, R₁₆, and R₁₇ is a halogen;
      B) a solvent; and
      C) an inorganic base; and
   II) reacting the mixture.

In one aspect, provided herein is a compound of Formula II-A, wherein 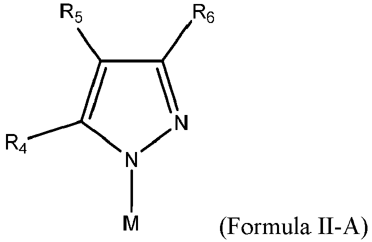
M is selected from alkali metals and alkaline metals;
each of R₄, R₅, and R₆ is independently selected from hydrogen and halogen; and
wherein at least one of R₄, R₅, and R₆ is hydrogen.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of' is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R₄, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, NBS, and 1,3-dibromo-5,5-dimethylhylhydantoin.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

Certain compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The embodiments of this disclosure include:
Embodiment 1. A method of preparing a compound of Formula VI, wherein 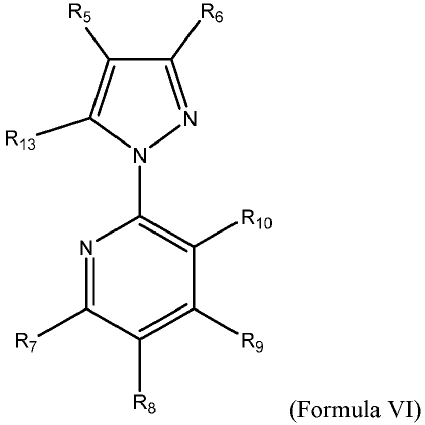
   each of R₅ - R₁₀ is independently selected from hydrogen and halogen; and
   R₁₃ is an organic acid, the method comprising
      I) forming a mixture comprising
         A) a compound of Formula V, wherein 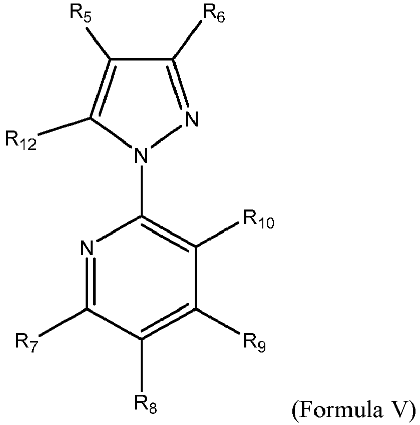
            each of R₅ - R₁₀ is independently selected from hydrogen and halogen;
            R₁₂ is selected from ether, ester, and nitrile; and
            wherein the compound of Formula V is prepared according to a method comprising
               i) forming a mixture comprising
                  a) a compound of Formula III, wherein 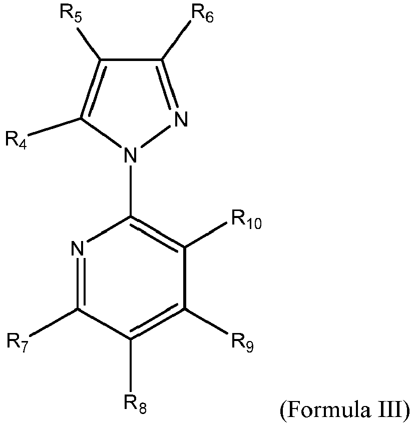
                     R₄ is hydrogen;
                     each of R₅ - R₁₀ is independently selected from hydrogen and halogen; and
                     wherein the compound of Formula III is prepared according to a method comprising
                        IA) forming a mixture comprising
                           AA) a compound of Formula II, wherein 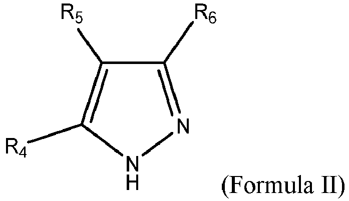
                              each of R₄, R₅, and R₆ is independently selected from hydrogen and halogen;
                              wherein at least one of R₄, R₅, and R₆ is hydrogen; and
                              wherein the compound of Formula II is prepared according to a method comprising
                                 ia) forming a mixture comprising
                                    aa) a compound of Formula VIII, wherein 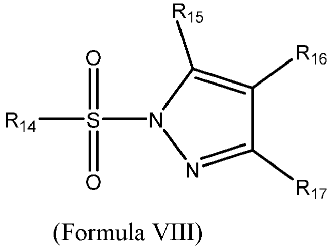
                                       R₁₄ is selected from substituted or unsubstituted aryl, substituted or unsubstituted heteroaryl, substituted or unsubstituted carbocycle, and substituted or unsubstituted heterocycle;
                                       each of R₁₅, R₁₆, and R₁₇ is independently selected from hydrogen and halogen; and
                                       wherein at least one of R₁₅, R₁₆, and R₁₇ is a halogen;
                                    bb) a solvent; and
                                    cc) an inorganic base; and
                                 iia) reacting the mixture;
                           BB) a compound of Formula IV, wherein 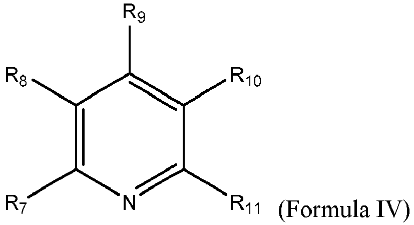 each of R₇ - R₁₁ is independently selected from hydrogen and halogen;
                           CC) a solvent;
                           DD) an inorganic base; and
                           EE) optionally an additive; and
                        IIA) reacting the mixture;
                           b) a solvent;
                           c) an organic compound;
                           d) a compound comprising a metal; and
                           e) optionally an additive; and
               ii) reacting the mixture; and
            B) a metal hydroxide; and
      II) reacting the mixture.
Embodiment 2. The method of embodiment 1, wherein the metal hydroxide is selected from alkali hydroxide, alkaline earth metal hydroxide, and combinations thereof.
Embodiment 3. The method of embodiment 2, wherein the alkali hydroxide is selected from lithium hydroxide, sodium hydroxide, and potassium hydroxide.
Embodiment 4. The method of embodiment 2, wherein the alkaline earth metal hydroxide is selected from calcium hydroxide and barium hydroxide.
Embodiment 5. The method of embodiment 1, wherein the method step II) of reacting the mixture occurs at a reaction temperature in the range of about 0 °C to about 90 °C.
Embodiment 6. The method of embodiment 1, wherein the compound comprising a metal is selected from a Grignard reagent and a lithium-containing compound.
Embodiment 7. The method of embodiment 6, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 8. The method of embodiment 7, wherein the Grignard reagent is *i*Pr₂NMgCl.
Embodiment 9. The method of embodiment 6, wherein the lithium-containing compound is selected from LDA, nBuLi, and combinations thereof.
Embodiment 10. The method of embodiment 1, wherein the solvent b) is selected from THF, toluene, 1,4-dioxane, Me-THF, and combinations thereof.
Embodiment 11. The method of embodiment 10, wherein the solvent b) is THF.
Embodiment 12. The method of embodiment 1, wherein the organic compound is selected from dimethyl carbonate, N,N-dimethyacetamide, and combinations thereof.
Embodiment 13. The method of embodiment 12, wherein the organic compound is dimethyl carbonate.
Embodiment 14. The method of embodiment 1, wherein the method step ii) of reacting the mixture occurs at a reaction temperature in the range of about 0 °C to about 60 °C.
Embodiment 15. The method of embodiment 14, wherein the method step ii) of reacting the mixture occurs at a reaction temperature in the range of about 0 °C to about 30 °C.
Embodiment 16. The method of embodiment 1, wherein R₅ and R₆ of Formula III are each independently hydrogen.
Embodiment 17. The method of embodiment 1, wherein the inorganic base DD) is selected from powder sodium hydroxide, powder potassium hydroxide, potassium carbonate, potassium phosphate, powder sodium methoxide, powder potassium t-butoxide, and combinations thereof.
Embodiment 18. The method of embodiment 1, wherein the solvent CC) is selected from toluene, N,N-dimethylformamide, N,N-dimethylacetamide, N-methyl-2-pyrrolidone, acetonitrile, and combinations thereof.
Embodiment 19. The method of embodiment 1, wherein the additive EE) is selected from potassium iodide, a phase transfer catalyst, and combinations thereof.
Embodiment 20. The method of embodiment 19, wherein the phase transfer catalyst is selected from butyl ammonium chloride, tetra butyl ammonium bromide, aliquat-336, 18-crown-6, and combinations thereof.
Embodiment 21. The method of embodiment 1, wherein the method step IIA) of reacting the mixture occurs at a reaction temperature in the range of about 100 °C to about 200 °C.
Embodiment 22. The method of embodiment 1, wherein the solvent bb) is selected from THF, toluene, heptanes, Me-THF, and combinations thereof.
Embodiment 23. The method of embodiment 1, wherein the inorganic base cc) is selected from sodium hydroxide, potassium hydroxide, sodium carbonate, potassium carbonate, and combinations thereof.
Embodiment 24. The method of embodiment 1, wherein the method step iia) of reacting the mixture occurs at a reaction temperature in the range of about 20 °C to about 150 °C.
Embodiment 25. The method of embodiment 1, wherein the compound of Formula VIII is prepared according to a method comprising
   I) forming a mixture comprising
      A) a compound of Formula VII, wherein 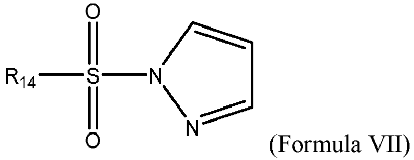 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 26. The method of embodiment 25, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 27. The method of embodiment 25, wherein the solvent is selected from THF, toluene, 1,4-dioxane, Me-THF, and combinations thereof.
Embodiment 28. The method of embodiment 25, wherein the compound comprising a metal is selected from a Grignard reagent and a lithium-containing compound.
Embodiment 29. The method of embodiment 28, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 30. The method of embodiment 28, wherein the lithium-containing compound is selected from LDA, nBuLi, and combinations thereof.
Embodiment 31. The method of embodiment 30, wherein the lithium-containing compound is nBuLi.
Embodiment 32. The method of embodiment 25, wherein the method step of reacting the mixture occurs at a reaction temperature in the range of about -78 °C to about 0 °C.
Embodiment 33. The method of embodiment 32, wherein the method step of reacting the mixture occurs at a reaction temperature in the range of about -65 °C to about -50 °C.
Embodiment 34. The method of embodiment 25, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 35. The method of embodiment 34, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 36. The method of embodiment 34, wherein the solvent is selected from toluene, xylene, heptanes, and combinations thereof.
Embodiment 37. The method of embodiment 34, wherein the base is selected from pyridine, triethylamine, sodium hydroxide, sodium hydroxide solution, powder sodium acetate, and combinations thereof.
Embodiment 38. The method of embodiment 34, wherein the method step of reacting the mixture occurs at a reaction temperature in the range of about 20 °C to about 150 °C.
Embodiment 39. A method of preparing a compound of Formula VI, wherein 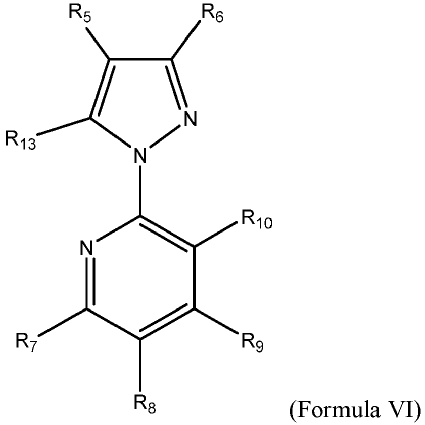
   each of R₅ - R₁₀ is independently selected from hydrogen and halogen; and
   R₁₃ is an organic acid, the method comprising
      I) forming a mixture comprising
         A) a compound of Formula III, wherein 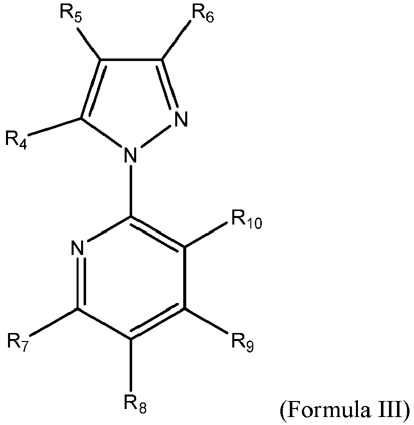
            R₄ is hydrogen;
            each of R₅ - R₁₀ is independently selected from hydrogen and halogen; and
            wherein the compound of Formula III is prepared according to a method comprising
               i) forming a mixture comprising
                  a) a compound of Formula II, wherein 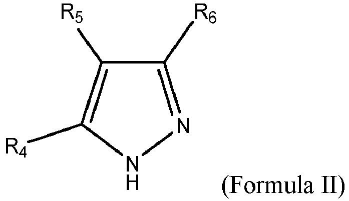
                     each of R₄, R₅, and R₆ is independently selected from hydrogen and halogen;
                     wherein at least one of R₄, R₅, and R₆ is hydrogen; and
                     wherein the compound of Formula II is prepared according to a method comprising
                        IA) forming a mixture comprising
                           AA) a compound of Formula VIII, wherein 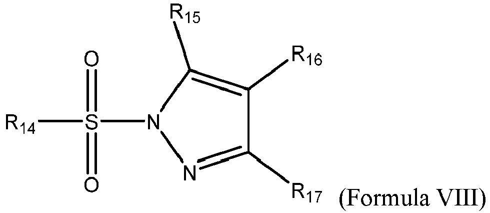
                              R₁₄ is selected from substituted or unsubstituted aryl, substituted or unsubstituted heteroaryl, substituted or unsubstituted carbocycle, and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           BB) a solvent; and
                           CC) an inorganic base; and
                        IIa) reacting the mixture;
                  b) a compound of Formula IV, wherein 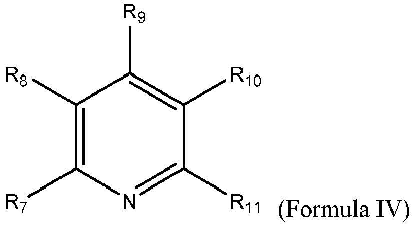 each of R₇ - R₁₁ is independently selected from hydrogen and halogen;
                  c) a solvent;
                  d) an inorganic base; and
                  e) optionally an additive; and
               ii) reacting the mixture;
         B) a carbonyl-containing compound;
         C) a solvent;
         D) a compound comprising a metal; and
         E) optionally an additive; and
   II) reacting the mixture.
Embodiment 40. The method of embodiment 39, wherein the compound comprising a metal is selected from a Grignard reagent and a lithium-containing compound.
Embodiment 41. The method of embodiment 40, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 42. The method of embodiment 41, wherein the Grignard reagent is *i*Pr₂NMgCl.
Embodiment 43. The method of embodiment 40, wherein the lithium-containing compound is selected from LDA, nBuLi, and combinations thereof.
Embodiment 44. The method of embodiment 39, wherein the solvent C) is selected from THF, toluene, 1,4-dioxane, Me-THF, and combinations thereof.
Embodiment 45. The method of embodiment 44, wherein the solvent C) is THF.
Embodiment 46. The method of embodiment 39, wherein the carbonyl-containing compound is selected from dimethylcarbonate, N,N-dimethylacetamide, carbon dioxide, and combinations thereof.
Embodiment 47. The method of embodiment 46, wherein the carbonyl-containing compound is carbon dioxide.
Embodiment 48. The method of embodiment 39, wherein the method step II) of reacting the mixture occurs at a reaction temperature in the range of about 0 °C to about 60 °C.
Embodiment 49. The method of embodiment 48, wherein the method step II) of reacting the mixture occurs at a reaction temperature in the range of about 0 °C to about 30 °C.
Embodiment 50. The method of embodiment 39, wherein the inorganic base d) is selected from powder sodium hydroxide, powder potassium hydroxide, potassium carbonate, potassium phosphate, powder sodium methoxide, powder potassium t-butoxide, and combinations thereof.
Embodiment 51. The method of embodiment 39, wherein the solvent c) is selected from toluene, N,N-dimethylformamide, N,N-dimethylacetamide, N-methyl-2-pyrrolidone, acetonitrile, and combinations thereof.
Embodiment 52. The method of embodiment 39, wherein the additive e) is selected from potassium iodide, a phase transfer catalyst, and combinations thereof.
Embodiment 53. The method of embodiment 52, wherein the phase transfer catalyst is selected from butyl ammonium chloride, tetra butyl ammonium bromide, aliquat-336, 18-crown-6, and combinations thereof.
Embodiment 54. The method of embodiment 39, wherein the method step ii) of reacting the mixture occurs at a reaction temperature in the range of about 100 °C to about 200 °C.
Embodiment 55. The method of embodiment 39, wherein the solvent BB) is selected from THF, toluene, heptanes, Me-THF, and combinations thereof.
Embodiment 56. The method of embodiment 39, wherein the inorganic base CC) is selected from sodium hydroxide, potassium hydroxide, sodium carbonate, potassium carbonate, and combinations thereof.
Embodiment 57. The method of embodiment 39, wherein the method step IIa) of reacting the mixture occurs at a reaction temperature in the range of about 20 °C to about 150 °C.
Embodiment 58. The method of embodiment 39, wherein the compound of Formula VIII is prepared according to a method comprising
   I) forming a mixture comprising
      A) a compound of Formula VII, wherein 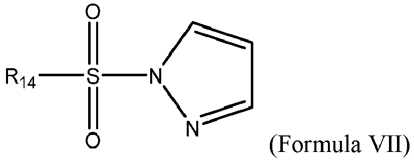 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 59. The method of embodiment 58, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 60. The method of embodiment 58, wherein the solvent is selected from THF, toluene, 1,4-dioxane, Me-THF, and combinations thereof.
Embodiment 61. The method of embodiment 58, wherein the compound comprising a metal is selected from a Grignard reagent and a lithium-containing compound.
Embodiment 62. The method of embodiment 61, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 63. The method of embodiment 61, wherein the lithium-containing compound is selected from LDA, nBuLi, and combinations thereof.
Embodiment 64. The method of embodiment 63, wherein the lithium-containing compound is nBuLi.
Embodiment 65. The method of embodiment 58, wherein the method step of reacting the mixture occurs at a reaction temperature in the range of about -78 °C to about 0 °C.
Embodiment 66. The method of embodiment 65, wherein the method step of reacting the mixture occurs at a reaction temperature in the range of about -65 °C to about -50 °C.
Embodiment 67. The method of embodiment 58, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 68. The method of embodiment 67, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 69. The method of embodiment 67, wherein the solvent is selected from toluene, xylene, heptanes, and combinations thereof.
Embodiment 70. The method of embodiment 67, wherein the base is selected from pyridine, triethylamine, sodium hydroxide, sodium hydroxide solution, powder sodium acetate, and combinations thereof.
Embodiment 71. The method of embodiment 67, wherein the method step of reacting the mixture occurs at a reaction temperature in the range of about 20 °C to about 150 °C.
Embodiment 72. A method of preparing a compound of Formula II, wherein 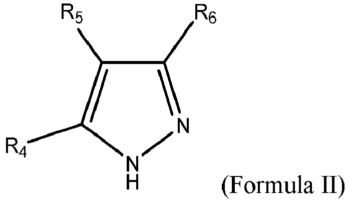
   each of R₄, R₅, and R₆ is independently selected from hydrogen and halogen; and
   wherein at least one of R₄, R₅, and R₆ is hydrogen, the method comprising
      I) forming a mixture comprising
         A) a compound of Formula VIII, wherein 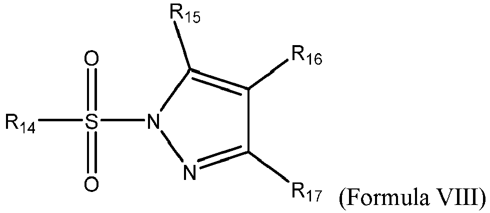
            R₁₄ is selected from substituted or unsubstituted aryl, substituted or unsubstituted heteroaryl, substituted or unsubstituted carbocycle, and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
         B) a solvent; and
         C) an inorganic base; and
      II) reacting the mixture.
Embodiment 73. The method of embodiment 72, wherein the solvent is selected from THF, toluene, heptanes, Me-THF, and combinations thereof.
Embodiment 74. The method of embodiment 72, wherein the inorganic base is selected from sodium hydroxide, potassium hydroxide, sodium carbonate, potassium carbonate, and combinations thereof.
Embodiment 75. The method of embodiment 72, wherein the method step of reacting the mixture occurs at a reaction temperature in the range of about 20 °C to about 150 °C.
Embodiment 76. The method of embodiment 72, wherein the compound of Formula VIII is prepared according to a method comprising
   I) forming a mixture comprising
      A) a compound of Formula VII, wherein 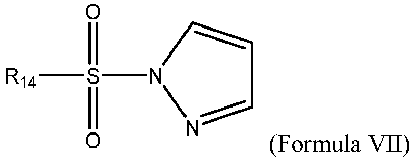 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 77. The method of embodiment 76, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 78. The method of embodiment 76, wherein the solvent is selected from THF, toluene, 1,4-dioxane, Me-THF, and combinations thereof.
Embodiment 79. The method of embodiment 76, wherein the compound comprising a metal is selected from a Grignard reagent and a lithium-containing compound.
Embodiment 80. The method of embodiment 79, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 81. The method of embodiment 79, wherein the lithium-containing compound is selected from LDA, nBuLi, and combinations thereof.
Embodiment 82. The method of embodiment 81, wherein the lithium-containing compound is nBuLi.
Embodiment 83. The method of embodiment 76, wherein the method step of reacting the mixture occurs at a reaction temperature in the range of about -78 °C to about 0 °C.
Embodiment 84. The method of embodiment 83, wherein the method step of reacting the mixture occurs at a reaction temperature in the range of about -65 °C to about -50 °C.
Embodiment 85. The method of embodiment 76, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 86. The method of embodiment 85, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 87. The method of embodiment 85, wherein the solvent is selected from toluene, xylene, heptanes, and combinations thereof.
Embodiment 88. The method of embodiment 85, wherein the base is selected from pyridine, triethylamine, sodium hydroxide, sodium hydroxide solution, powder sodium acetate, and combinations thereof.
Embodiment 89. The method of embodiment 85, wherein the method step of reacting the mixture occurs at a reaction temperature in the range of about 20 °C to about 150 °C.
Embodiment 90. A method of preparing a compound of Formula VII, wherein 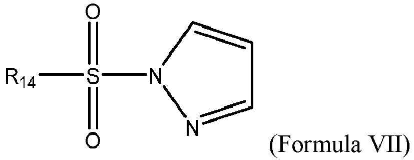 R₁₄ is selected from substituted or unsubstituted aryl, substituted or unsubstituted heteroaryl, substituted or unsubstituted carbocycle, and substituted or unsubstituted heterocycle, the method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 91. The method of embodiment 90, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 92. The method of embodiment 90, wherein the solvent is selected from toluene, xylene, heptanes, and combinations thereof.
Embodiment 93. The method of embodiment 90, wherein the base is selected from pyridine, triethylamine, sodium hydroxide, sodium hydroxide solution, powder sodium acetate, and combinations thereof.
Embodiment 94. The method of embodiment 90, wherein the method step of reacting the mixture occurs at a reaction temperature in the range of about 20 °C to about 150 °C.
Embodiment 95. A method of preparing a compound of Formula VIII, wherein 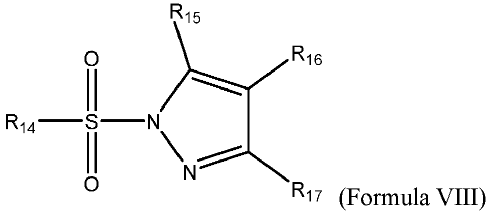
   R₁₄ is selected from substituted or unsubstituted aryl, substituted or unsubstituted heteroaryl, substituted or unsubstituted carbocycle, and substituted or unsubstituted heterocycle;
   each of R₁₅, R₁₆, and R₁₇ is independently selected from hydrogen and halogen; and
   wherein at least one of R₁₅, R₁₆, and R₁₇ is a halogen, the method comprising
      I) forming a mixture comprising
         A) the compound of Formula VII, wherein 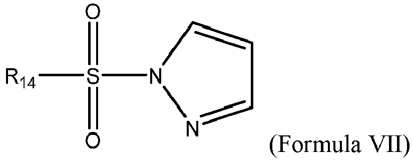 R₁₄ is selected from substituted or unsubstituted aryl, substituted or unsubstituted heteroaryl, substituted or unsubstituted carbocycle, and substituted or unsubstituted heterocycle;
         B) a halogenation reagent;
         C) a solvent; and
         D) a compound comprising a metal; and
      II) reacting the mixture.
Embodiment 96. The method of embodiment 95, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 97. The method of embodiment 90, wherein the solvent is selected from toluene, xylene, heptanes, and combinations thereof.
Embodiment 98. The method of embodiment 95, wherein the compound comprising a metal is selected from a Grignard reagent and a lithium-containing compound.
Embodiment 99. The method of embodiment 98, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 100. The method of embodiment 98, wherein the lithium-containing compound is selected from LDA, nBuLi, and combinations thereof.
Embodiment 101. The method of embodiment 100, wherein the lithium-containing compound is nBuLi.
Embodiment 102. The method of embodiment 95, wherein the method step of reacting the mixture occurs at a reaction temperature in the range of about -78 °C to about 0 °C.
Embodiment 103. The method of embodiment 102, wherein the method step of reacting the mixture occurs at a reaction temperature in the range of about -65 °C to about -50 °C.
Embodiment 104. The method of embodiment 95, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 105. The method of embodiment 104, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 106. The method of embodiment 104, wherein the solvent is selected from toluene, xylene, heptanes, and combinations thereof.
Embodiment 107. The method of embodiment 104, wherein the base is selected from pyridine, triethylamine, sodium hydroxide, sodium hydroxide solution, powder sodium acetate, and combinations thereof.
Embodiment 108. The method of embodiment 104, wherein the method step of reacting the mixture occurs at a reaction temperature in the range of about 20 °C to about 150 °C.
Embodiment 109. A method of preparing a compound of Formula V, wherein 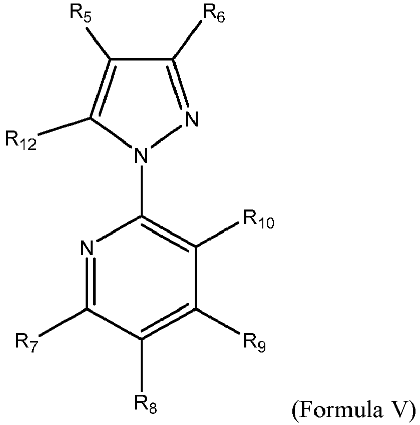
   each of R₅ - R₁₀ is independently selected from hydrogen and halogen; and
   R₁₂ is selected from ether, ester, and nitrile, the method comprising
      i) forming a mixture comprising
         a) a compound of Formula III, wherein 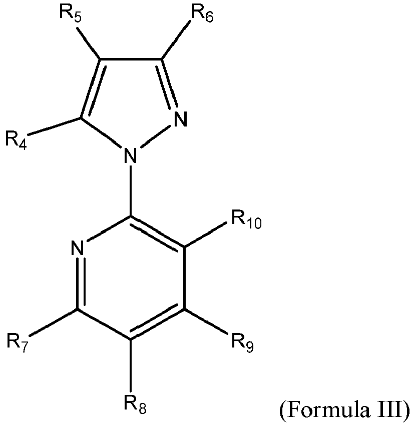
            R₄ is hydrogen;
            each of R₅ - R₁₀ is independently selected from hydrogen and halogen; and
            wherein the compound of Formula III is prepared according to a method comprising
               IA) forming a mixture comprising
                  AA) a compound of Formula II, wherein 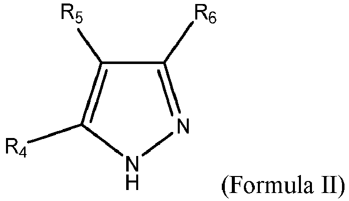
                     each of R₄, R₅, and R₆ is independently selected from hydrogen and halogen;
                     wherein at least one of R₄, R₅, and R₆ is hydrogen; and
                     wherein the compound of Formula II is prepared according to a method comprising
                        ia) forming a mixture comprising
                           aa) a compound of Formula VIII, wherein 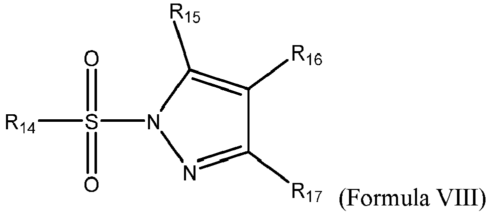
                              R₁₄ is selected from substituted or unsubstituted aryl, substituted or unsubstituted heteroaryl, substituted or unsubstituted carbocycle, and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           bb) a solvent; and
                           cc) an inorganic base; and
                        iia) reacting the mixture;
                     BB) a compound of Formula IV, wherein 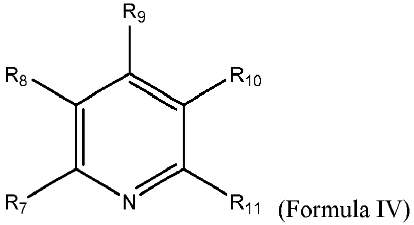 each of R₇ - R₁₁ is independently selected from hydrogen and halogen;
                     CC) a solvent;
                     DD) an inorganic base; and
                     EE) optionally an additive; and
                  IIA) reacting the mixture;
         b) a solvent;
         c) an organic compound;
         d) a compound comprising a metal; and
         e) optionally an additive; and
      ii) reacting the mixture.
Embodiment 110. The method of embodiment 109, wherein the compound comprising a metal is selected from a Grignard reagent and a lithium-containing compound.
Embodiment 111. The method of embodiment 110, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 112. The method of embodiment 111, wherein the Grignard reagent is *i*Pr₂NMgCl.
Embodiment 113. The method of embodiment 110, wherein the lithium-containing compound is selected from LDA, nBuLi, and combinations thereof.
Embodiment 114. The method of embodiment 109, wherein the solvent b) is selected from THF, toluene, 1,4-dioxane, Me-THF, and combinations thereof.
Embodiment 115. The method of embodiment 114, wherein the solvent b) is THF.
Embodiment 116. The method of embodiment 109, wherein the organic compound is selected from dimethyl carbonate, N,N-dimethyacetamide, and combinations thereof.
Embodiment 117. The method of embodiment 109, wherein the organic compound is dimethyl carbonate.
Embodiment 118. The method of embodiment 109, wherein the method step ii) of reacting the mixture occurs at a reaction temperature in the range of about 0 °C to about 60 °C.
Embodiment 119. The method of embodiment 118, wherein the method step ii) of reacting the mixture occurs at a reaction temperature in the range of about 0 °C to about 30 °C.
Embodiment 120. The method of embodiment 109, wherein R₅ and R₆ of Formula III are each independently hydrogen.
Embodiment 121. The method of embodiment 109, wherein the inorganic base DD) is selected from powder sodium hydroxide, powder potassium hydroxide, potassium carbonate, potassium phosphate, powder sodium methoxide, powder potassium *t*-butoxide, and combinations thereof.
Embodiment 122. The method of embodiment 109, wherein the solvent CC) is selected from toluene, N,N-dimethylformamide, N,N-dimethylacetamide, N-methyl-2-pyrrolidone, acetonitrile, and combinations thereof.
Embodiment 123. The method of embodiment 109, wherein the additive EE) is selected from potassium iodide, a phase transfer catalyst, and combinations thereof.
Embodiment 124. The method of embodiment 123, wherein the phase transfer catalyst is selected from butyl ammonium chloride, tetra butyl ammonium bromide, aliquat-336, 18-crown-6, and combinations thereof.
Embodiment 125. The method of embodiment 109, wherein the method step IIA) of reacting the mixture occurs at a reaction temperature in the range of about 100 °C to about 200 °C.
Embodiment 126. The method of embodiment 109, wherein the solvent bb) is selected from THF, toluene, heptanes, Me-THF, and combinations thereof.
Embodiment 127. The method of embodiment 109, wherein the inorganic base cc) is selected from sodium hydroxide, potassium hydroxide, sodium carbonate, potassium carbonate, and combinations thereof.
Embodiment 128. The method of embodiment 109, wherein the method step iia) of reacting the mixture occurs at a reaction temperature in the range of about 20 °C to about 150 °C.
Embodiment 129. The method of embodiment 109, wherein the compound of Formula VIII is prepared according to a method comprising
   I) forming a mixture comprising
      A) a compound of Formula VII, wherein 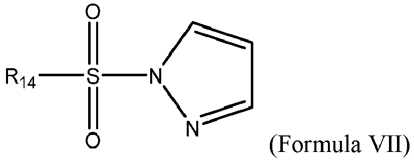 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 130. The method of embodiment 129, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 131. The method of embodiment 129, wherein the solvent is selected from THF, toluene, 1,4-dioxane, Me-THF, and combinations thereof.
Embodiment 132. The method of embodiment 129, wherein the compound comprising a metal is selected from a Grignard reagent and a lithium-containing compound.
Embodiment 133. The method of embodiment 132, wherein the Grignard reagent is selected from MeMgCl, *i*PrMgCl, *i*PrMgBr, EtMgCl, *i*Pr₂NMgCl, *i*Pr₂NMgBr, Et₂NMgCl, TMPMgCl, TMPM_{G}Cl•LiCl, *i*Pr₂NMgCl•LiCl, *i*Pr₂NMgBr•LiCl, and combinations thereof.
Embodiment 134. The method of embodiment 132, wherein the lithium-containing compound is selected from LDA, nBuLi, and combinations thereof.
Embodiment 135. The method of embodiment 134, wherein the lithium-containing compound is nBuLi.
Embodiment 136. The method of embodiment 129, wherein the method step of reacting the mixture occurs at a reaction temperature in the range of about -78 °C to about 0 °C.
Embodiment 137. The method of embodiment 136, wherein the method step of reacting the mixture occurs at a reaction temperature in the range of about -65 °C to about -50 °C.
Embodiment 138. The method of embodiment 129, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 139. The method of embodiment 138, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 140. The method of embodiment 138, wherein the solvent is selected from toluene, xylene, heptanes, and combinations thereof.
Embodiment 141. The method of embodiment 138, wherein the base is selected from pyridine, triethylamine, sodium hydroxide, sodium hydroxide solution, powder sodium acetate, and combinations thereof.
Embodiment 142. The method of embodiment 138, wherein the method step of reacting the mixture occurs at a reaction temperature in the range of about 20 °C to about 150 °C.
Embodiment 143. A method of preparing a compound of Formula III, wherein 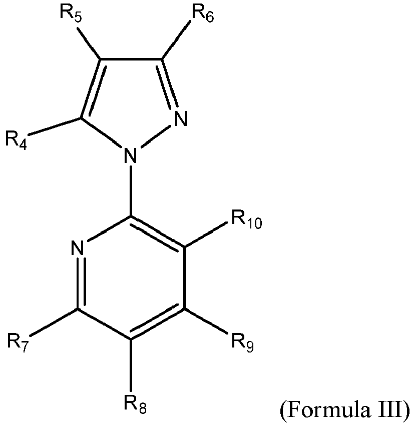
   R₄ is hydrogen; and
   each of R₅ - R₁₀ is independently selected from hydrogen and halogen, the method comprising
      i) forming a mixture comprising
         a) a compound of Formula II, wherein 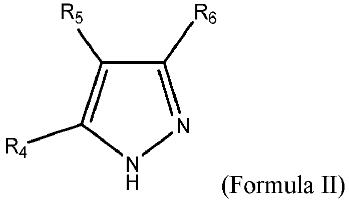
            each of R₄, R₅, and R₆ is independently selected from hydrogen and halogen;
            wherein at least one of R₄, R₅, and R₆ is hydrogen; and
            wherein the compound of Formula II is prepared according to a method comprising
               IA) forming a mixture comprising
                  AA) a compound of Formula VIII, wherein 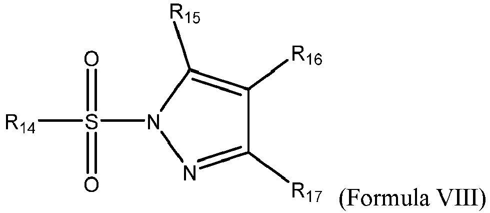
                     R₁₄ is selected from substituted or unsubstituted aryl, substituted or unsubstituted heteroaryl, substituted or unsubstituted carbocycle, and substituted or unsubstituted heterocycle;
                     each of R₁₅, R₁₆, and R₁₇ is independently selected from hydrogen and halogen; and
                     wherein at least one of R₁₅, R₁₆, and R₁₇ is a halogen;
                  BB) a solvent; and
                  CC) an inorganic base; and
               IIa) reacting the mixture;
         b) a compound of Formula IV, wherein 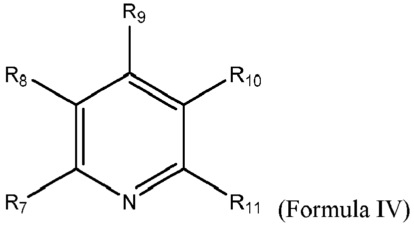 each of R₇ - R₁₁ is independently selected from hydrogen and halogen;
         c) a solvent;
         d) an inorganic base; and
         e) optionally an additive; and
   ii) reacting the mixture.
Embodiment 144. The method of embodiment 143, wherein R₅ and R₆ of Formula III are each independently hydrogen.
Embodiment 145. The method of embodiment 143, wherein the inorganic base d) is selected from powder sodium hydroxide, powder potassium hydroxide, potassium carbonate, potassium phosphate, powder sodium methoxide, powder potassium t-butoxide, and combinations thereof.
Embodiment 146. The method of embodiment 143, wherein the solvent c) is selected from toluene, N,N-dimethylformamide, N,N-dimethylacetamide, N-methyl-2-pyrrolidone, acetonitrile, and combinations thereof.
Embodiment 147. The method of embodiment 143, wherein the additive e) is selected from potassium iodide, a phase transfer catalyst, and combinations thereof.
Embodiment 148. The method of embodiment 147, wherein the phase transfer catalyst is selected from butyl ammonium chloride, tetra butyl ammonium bromide, aliquat-336, 18-crown-6, and combinations thereof.
Embodiment 149. The method of embodiment 143, wherein the method step ii) of reacting the mixture occurs at a reaction temperature in the range of about 100 °C to about 200 °C.
Embodiment 150. The method of embodiment 143, wherein the solvent BB) is selected from THF, toluene, heptanes, Me-THF, and combinations thereof.
Embodiment 151. The method of embodiment 143, wherein the inorganic base CC) is selected from sodium hydroxide, potassium hydroxide, sodium carbonate, potassium carbonate, and combinations thereof.
Embodiment 152. The method of embodiment 143, wherein the method step IIa) of reacting the mixture occurs at a reaction temperature in the range of about 20 °C to about 150 °C.
Embodiment 153. The method of embodiment 143, wherein the compound of Formula VIII is prepared according to a method comprising
   I) forming a mixture comprising
      A) a compound of Formula VII, wherein 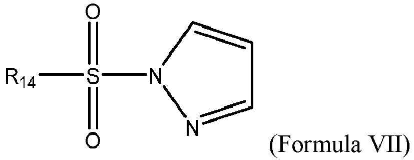 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 154. The method of embodiment 153, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 155. The method of embodiment 153, wherein the solvent is selected from THF, toluene, 1,4-dioxane, Me-THF, and combinations thereof.
Embodiment 156. The method of embodiment 153, wherein the compound comprising a metal is selected from a Grignard reagent and a lithium-containing compound.
Embodiment 157. The method of embodiment 156, wherein the Grignard reagent is selected from MeMgCl, *i*PrMgCl, *i*PrMgBr, EtMgCl, *i*Pr₂NMgCl, *i*Pr₂NMgBr, Et₂NMgCl, TMPMgCl, TMPM_{G}Cl•LiCl, *i*Pr₂NMgCl•LiCl, *i*Pr₂NMgBr•LiCl, and combinations thereof.
Embodiment 158. The method of embodiment 156, wherein the lithium-containing compound is selected from LDA, nBuLi, and combinations thereof.
Embodiment 159. The method of embodiment 158, wherein the lithium-containing compound is nBuLi.
Embodiment 160. The method of embodiment 153, wherein the method step of reacting the mixture occurs at a reaction temperature in the range of about -78 °C to about 0 °C.
Embodiment 161. The method of embodiment 160, wherein the method step of reacting the mixture occurs at a reaction temperature in the range of about -65 °C to about -50 °C.
Embodiment 162. The method of embodiment 153, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 163. The method of embodiment 162, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 164. The method of embodiment 162, wherein the solvent is selected from toluene, xylene, heptanes, and combinations thereof.
Embodiment 165. The method of embodiment 162, wherein the base is selected from pyridine, triethylamine, sodium hydroxide, sodium hydroxide solution, powder sodium acetate, and combinations thereof.
Embodiment 166. The method of embodiment 162, wherein the method step of reacting the mixture occurs at a reaction temperature in the range of about 20 °C to about 150 °C.
Embodiment 167. A method of preparing a compound of Formula II-A, wherein 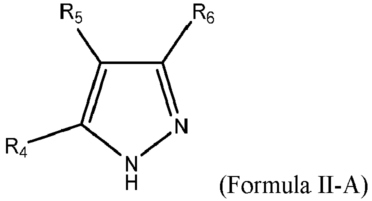
   M is selected from alkali metals and alkaline metals;
   each of R₄, R₅, and R₆ is independently selected from hydrogen and halogen; and
   wherein at least one of R₄, R₅, and R₆ is hydrogen, the method comprising
      I) forming a mixture comprising
         A) a compound of Formula VIII, wherein 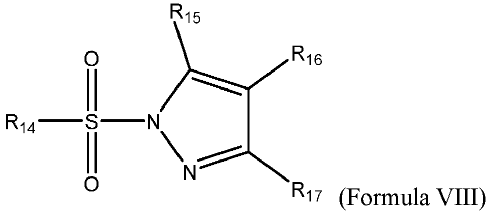
            R₁₄ is selected from substituted or unsubstituted aryl, substituted or unsubstituted heteroaryl, substituted or unsubstituted carbocycle, and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
         B) a solvent; and
         C) an inorganic base; and
      II) reacting the mixture.
Embodiment 168. The method of embodiment 167, wherein the solvent is selected from THF, toluene, heptanes, Me-THF, and combinations thereof.
Embodiment 169. The method of embodiment 167, wherein the inorganic base is selected from sodium hydroxide, potassium hydroxide, sodium carbonate, potassium carbonate, and combinations thereof.
Embodiment 170. The method of embodiment 167, wherein the method step of reacting the mixture occurs at a reaction temperature in the range of about 20 °C to about 150 °C.
Embodiment 171. The method of claim 167, wherein M is selected from lithium, sodium, potassium, calcium, and magnesium.
Embodiment 172. The method of embodiment 167, wherein the compound of Formula VIII is prepared according to a method comprising
   I) forming a mixture comprising
      A) a compound of Formula VII, wherein 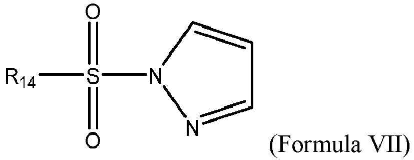 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 173. The method of embodiment 172, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 174. The method of embodiment 172, wherein the solvent is selected from THF, toluene, 1,4-dioxane, Me-THF, and combinations thereof.
Embodiment 175. The method of embodiment 172, wherein the compound comprising a metal is selected from a Grignard reagent and a lithium-containing compound.
Embodiment 176. The method of embodiment 172, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPM_{G}Cl•LiCl, *i*Pr₂NMgCl•LiCl, *i*Pr₂NMgBr•LiCl, and combinations thereof.
Embodiment 177. The method of embodiment 172, wherein the lithium-containing compound is selected from LDA, nBuLi, and combinations thereof.
Embodiment 178. The method of embodiment 177, wherein the lithium-containing compound is nBuLi.
Embodiment 179. The method of embodiment 172, wherein the method step of reacting the mixture occurs at a reaction temperature in the range of about -78 °C to about 0 °C.
Embodiment 180. The method of embodiment 179, wherein the method step of reacting the mixture occurs at a reaction temperature in the range of about -65 °C to about -50 °C.
Embodiment 181. The method of embodiment 172, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 182. The method of embodiment 181, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 183. The method of embodiment 181, wherein the solvent is selected from toluene, xylene, heptanes, and combinations thereof.
Embodiment 184. The method of embodiment 181, wherein the base is selected from pyridine, triethylamine, sodium hydroxide, sodium hydroxide solution, powder sodium acetate, and combinations thereof.
Embodiment 185. The method of embodiment 181, wherein the method step of reacting the mixture occurs at a reaction temperature in the range of about 20 °C to about 150 °C.
Embodiment 186. A compound of Formula II-A, wherein 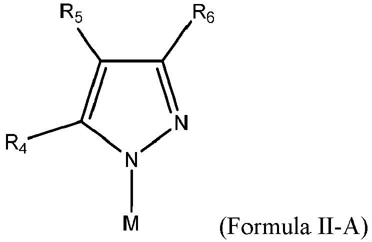
   M is selected from alkali metals and alkaline metals;
   each of R₄, R₅, and R₆ is independently selected from hydrogen and halogen; and
   wherein at least one of R₄, R₅, and R₆ is hydrogen.
Embodiment 187. The compound of embodiment 186, wherein the compound is

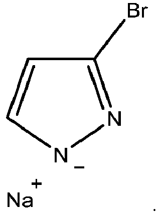

In one aspect, a compound of Formula VI is prepared according to a method represented by Scheme 1. The R groups are as defined anywhere in this disclosure.

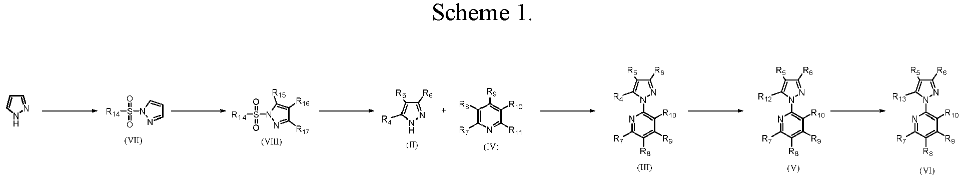

In one aspect, a compound of Formula VI is prepared according to a method represented by Scheme 2. The R groups are as defined anywhere in this disclosure.

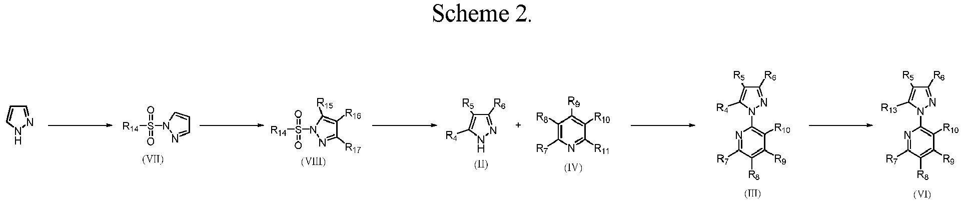

In one aspect, 5-Bromo-2-(3-chloro-pyridin-2-yl)-2H-pyrazole-3-carboxylic acid is prepared according to a method represented by Scheme 3.

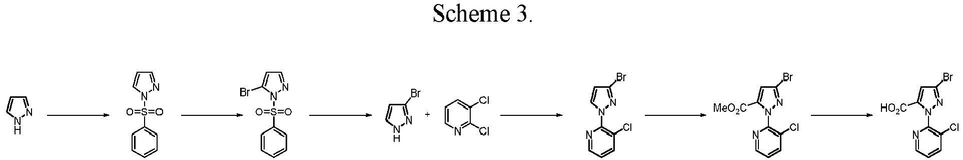

In one aspect, 5-Bromo-2-(3-chloro-pyridin-2-yl)-2H-pyrazole-3-carboxylic acid is prepared according to a method represented by Scheme 4.

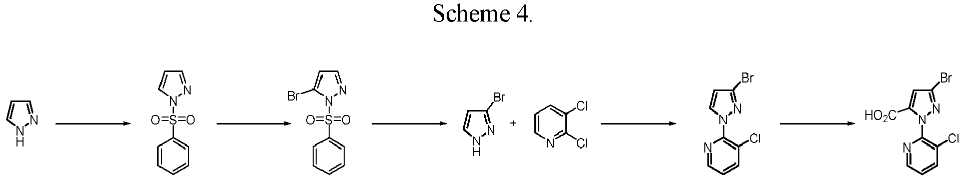

In one aspect, a compound of Formula VII is prepared according to a method represented by Scheme 9. The R groups are as defined anywhere in this disclosure.

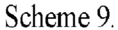

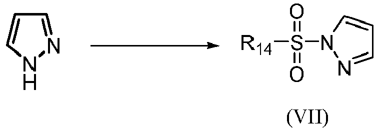

This aspect includes reacting pyrazole with a protective group reagent optionally in the presence of a base and optionally in an organic solvent. In one embodiment, the protective group reagent is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride, and combinations thereof. In another embodiment, the protective reagent is benzenesulfonyl chloride. In one embodiment, the organic solvent is selected from toluene, xylene, heptanes, or combinations thereof. In another embodiment, the organic solvent is toluene. In one embodiment, the base is selected from an organic base and an inorganic base. In another embodiment, the base is selected from pyridine, triethylamine, sodium hydroxide, sodium hydroxide solution, powder sodium acetate, and combinations thereof. In another embodiment, there is no base. In one embodiment, the reaction temperature is in the range from about 20 °C to about 150 °C. In another embodiment, the reaction temperature is in the range from about 50 °C to about 90 °C.

In one aspect, a compound of Formula VIII is prepared according to a method represented by Scheme 10. The R groups are as defined anywhere in this disclosure.

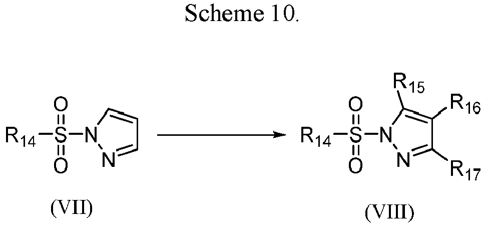

This aspect includes reacting a compound of Formula VII with a halogenation reagent in an organic solvent in the presence of a base reagent. In one embodiment, the halogenation reagent is selected from Br₂, N-Bromosuccinimide, 1,3-Dibromo-5,5-dimethylhylhydantoin, 1,2-dibromotetrachloroethane (DBTCE), and combinations thereof. In another embodiment, the halogenation reagent is Br₂. In one embodiment, the organic solvent is selected from THF, toluene, 1,4-dioxane, Me-THF, and combinations thereof. In another embodiment, the solvent is THF. In one embodiment, base reagent is selected from LDA, nBuLi, iPr₂NMgCl, *i*Pr₂NMgBr, Et₂NMgCl, TMPMgCl, TMPMgCI•LiCl, *i*Pr₂NMgCl•LiCl, *i*Pr₂NMgBr•LiCl, and combinations thereof. In another embodiment, the base is nBuLi. In one embodiment, the reaction temperature is in the range from about -78 °C to about 0 °C. In another embodiment, the reaction temperature is in the range from about -65 °C to about -50 °C.

In one aspect, a compound of Formula II is prepared according to a method represented by Scheme 11. The R groups are as defined anywhere in this disclosure.

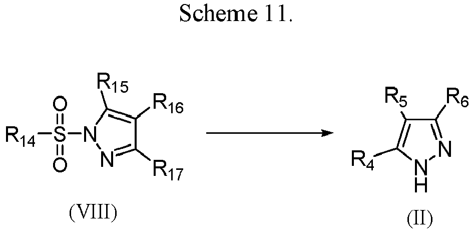

This aspect includes reacting a compound of Formula VIII with an organic solvent in the presence of an inorganic base. In one embodiment, the solvent is selected from THF, toluene, heptanes, Me-THF, and combinations thereof. In another embodiment, the solvent is toluene. In one embodiment, the inorganic base is selected from NaOH, KOH, Na₂CO₃, K₂CO₃, and combinations thereof. In another embodiment, the inorganic base is NaOH. In one embodiment, the reaction temperature is in the range from about 20 °C to about 150 °C. In another embodiment, the reaction temperature is in the range from about 50 °C to about 120 °C.

In one aspect, a compound of Formula II-A is prepared according to a method represented by Scheme 12. The R groups are as defined anywhere in this disclosure.

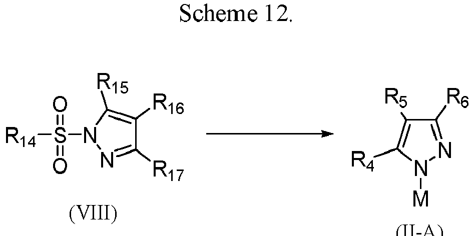

In one embodiment, the compound of Formula II-A is a metal salt of Formula II. In one embodiment, the bond between M and N is an ionic bond. In one embodiment, M is selected from alkali metals and alkaline metals. In one embodiment, M is selected from lithium, sodium, and potassium. In another embodiment, M is sodium. In another embodiment, M is selected from calcium and magnesium.

In one embodiment, the compound of Formula II-A is

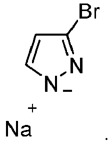

This aspect includes reacting a compound of Formula VIII with an organic solvent in the presence of an inorganic base. In one embodiment, the solvent is selected from THF, toluene, heptanes, Me-THF, and combinations thereof. In another embodiment, the solvent is toluene. In one embodiment, the inorganic base is selected from NaOH, KOH, Na₂CO₃, K₂CO₃, and combinations thereof. In another embodiment, the inorganic base is NaOH. In one embodiment, the reaction temperature is in the range from about 20 °C to about 150 °C. In another embodiment, the reaction temperature is in the range from about 50 °C to about 120 °C.

In one aspect, a compound of Formula III is prepared according to a method represented by Scheme 13. The R groups are as defined anywhere in this disclosure.

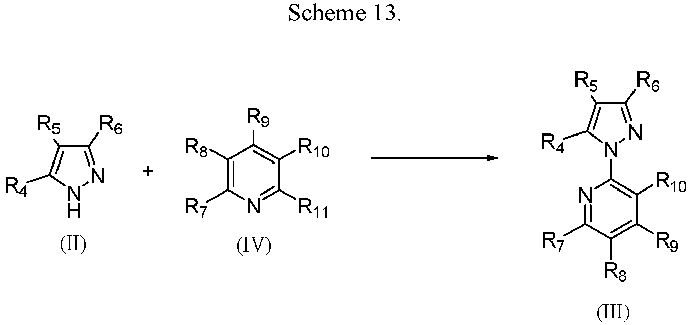

This aspect includes mixing a compound of Formula II with a compound of Formula IV in a solvent in the presence of an inorganic base and optionally an additive. In one embodiment, the inorganic base is selected from powder sodium hydroxide, powder potassium hydroxide, potassium carbonate, potassium phosphate powder sodium methoxide, powder potassium *t*-butoxide, and combinations thereof. In one embodiment, the solvent is selected from toluene, N,N-Dimethylformamide (DMF), N,N-Dimethylacetamide (DMAc), N-methyl-2-pyrrolidone (NMP), acetonitrile, and combinations thereof. In another embodiment, the solvent is toluene. In one embodiment, the reaction temperature is in the range from about 100 °C to about 200 °C. In another embodiment, the reaction temperature is in the range from about 130 °C to about 180 °C. In another embodiment, the temperature is in the range from about 145 °C to about 160 °C.

In one aspect, a compound of Formula VI is prepared according to a method represented by Scheme 14. The R groups are as defined anywhere in this disclosure.

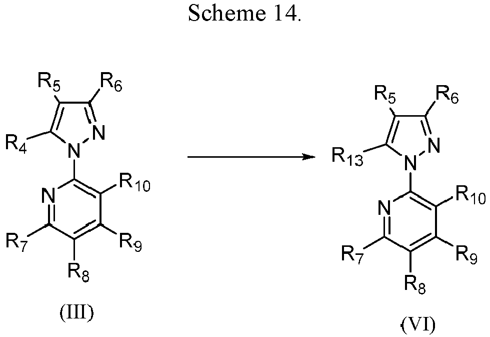

This aspect includes mixing a compound of Formula III with CO₂ in a solvent in the presence of a base reagent and optionally an additive. In one embodiment, the base reagent is selected from MeMgCl, *i*PrMgCl, *i*PrMgBr, EtMgCl, LDA, nBuLi, iPr₂NMgCl, *i*Pr₂NMgBr, Et₂NMgCl, TMPMgCl, TMPM_{G}Cl•LiCl, *i*Pr₂NMgCl•LiCl, *i*Pr₂NMgBr•LiCl, and combinations thereof. In another embodiment, the base reagent is *i*Pr₂NMgCl. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 60 °C. In another embodiment, the temperature is in the range from about 0 °C to about 30 °C.

In one aspect, a compound of Formula V is prepared according to a method represented by Scheme 15. The R groups are as defined anywhere in this disclosure.

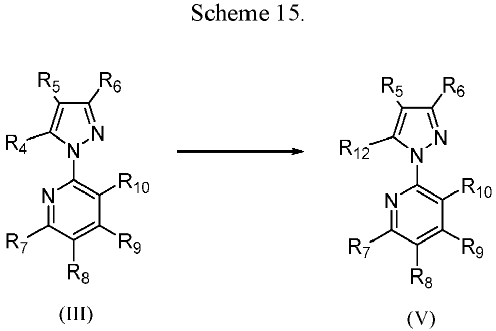

This aspect includes mixing a compound of Formula III with dimethyl carbonate (DMC) in a solvent in the presence of a base reagent and optionally an additive. In one embodiment, the organic base reagent is selected from MeMgCl, *i*PrMgCl, *i*PrMgBr, EtMgCl, LDA, nBuLi, *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the organic base is *i*Pr₂NMgCl. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 60 °C. In another embodiment, the temperature is in the range from about 0 °C to about 30 °C.

In one aspect, a compound of Formula VI is prepared according to a method represented by Scheme 16. The R groups are as defined anywhere in this disclosure.

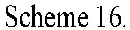

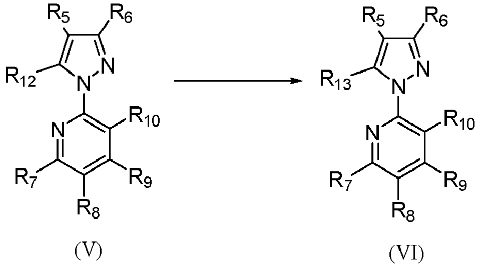

This aspect includes reacting a compound of Formula V with an aqueous metal hydroxide solution. In one embodiment, the metal hydroxide is selected from alkali hydroxide, alkaline earth metal hydroxide, and combinations thereof. In one embodiment, the alkali hydroxide is selected from lithium hydroxide, sodium hydroxide, potassium hydroxide, and combinations. In one embodiment, the alkaline earth metal hydroxide is selected from calcium hydroxide, barium hydroxide, and combinations thereof. In another embodiment, the metal hydroxide is sodium hydroxide or potassium hydroxide. In one embodiment, the reaction temperature is in the range from about 0 °C to about 90 °C. In another embodiment, the reaction temperature is in the range from about 60 °C to about 80 °C. In another embodiment, the reaction temperature is in the range from about 40 °C to about 70 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

### Example 1. Protecting reaction.

50 grams of pyrazole was dissolved in 150 g of toluene, and then 29.6 g of NaOH was charged and 136 g of benzenesulfonyl chloride was charged. The reaction was heated to reflux for 2 hours and then cooled to 25 °C. 100 g of 5% NaHCO₃ was charged, and stirred for 1 hour, and then the organic layer was separated and distilled to yield 147 g of Bz-protected pyrazole as product.

### Example 2. Halogenation.

50 grams of Bz-protected pyrazole was dissolved in 500 g of dry THF and this mixture was cooled to -50 °C. Next, 132 ml of BuLi (2.5 M in hexane) was added dropwise at a temperature below -50 °C. After addition, the reaction was stirred at -50 °C for 1 hour and then 73.6 g of dry Br₂ was added dropwise at -50 °C. After addition, the reaction was quenched by 15% HOAc and the organic layer was collected and distilled to yield 64 g of 5-bromo-1-(phenylsulfonyl)-1H-pyrazole.

### Example 3. Removal of protecting group.

5-bromo-1-(phenylsulfonyl)-1H-pyrazole was dissolved in toluene and 22 g of NaOH was charged. Next, the reaction was heated to reflux for 1 hour and then water was removed by azeotropic distillation. Then the mixture was cooled to 25 °C and used for subsequent steps.

### Example 4. Coupling reaction.

11.5 grams of 3-bromo-1H-pyrazole and 14.7 g of 40% NaOH solution were reacted in the presence of toluene and at a temperature of 150 to 160 °C. Water was removed by azetropic distillation under reflux temperature to yield the corresponding 3-bromo-1H-pyrazole sodium salt. Then, toluene and 13.5 g of 2,3-dichloropyridine were added and the mixture was reacted at 150-160 °C. After reaction, the reaction mixture was quenched with water and the organic layer was separated. The organic layer was then washed with water and concentrated under vacuum at 40-45 °C to obtain 2-(3-bromo-1H-pyrazol-1-yl)-3-chloropyridine as a solid.

### Example 5. Reaction in the presence of a Grignard reagent.

32 grams of 2-(3-bromo-1H-pyrazol-1-yl)-3-chloropyridine was dissolved in THF then iPr₂NMgCl (in situ MeMgCl and iPr₂NH) was added at 0 °C to yield the corresponding 2-(3-bromo-1H-pyrazol-1-yl)-3-chloropyridine magnesium salt. After 2.5 hours, 52.0 g of DMC was added drop-wise at room temperature over 6 hours. The reaction temperature was controlled at 20-40 °C. After reaction, THF and DMC were distilled off under reduced pressure, and then the reaction mixture was quenched with water. Next, toluene was added. After separation and concentration, 36.7 g of high purity (90%, LC Area) of methyl 3-bromo-1-(3-chloropyridin-2-yl)-1H-pyrazole-5-carboxylate was obtained.

### Example 6. Hydrolysis.

30 grams of methyl 3-bromo-1-(3-chloropyridin-2-yl)-1H-pyrazole-5-carboxylate and a caustic soda solution in 90 g of toluene were charged to a flask at 85°C. The the mixture was kept at 80-85 °C for 2 hours to complete reaction. The aqueous phase was washed with toluene twice. Dilute H₂SO₄ was used to adjust pH to about 1. After filtration and drying, 26.5 g (97%, LC Area) of 5-bromo-2-(3-chloro-pyridin-2-yl)-2H-pyrazole-3-carboxylic acid was obtained.

### Example 7. Carboxylation.

32 grams of 2-(3-bromo-1H-pyrazol-1-yl)-3-chloropyridine was dissolved in THF and then iPr₂NMgCl, obtained in situ by separately adding MeMgCl and iPr₂NH, was added at 0 °C to yield the corresponding 2-(3-bromo-1H-pyrazol-1-yl)-3-chloropyridine magnesium salt. After 2.5 hours, CO₂ gas was added at room temperature over 1 hour. The reaction temperature was controlled at 20-40 °C. After reaction, THF was distilled off under reduced pressure, and then the reaction mixture was quenched with water. Next, toluene was added. After separation and concentration, 30.0 g of high purity (95%, LC Area) of 5-bromo-2-(3-chloro-pyridin-2-yl)-2H-pyrazole-3-carboxylic acid was obtained.

This written description uses examples to illustrate the present disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

Further aspects of the invention (Aspects A1-A20) are as follows:
A1. A method of preparing a compound of Formula VI, wherein 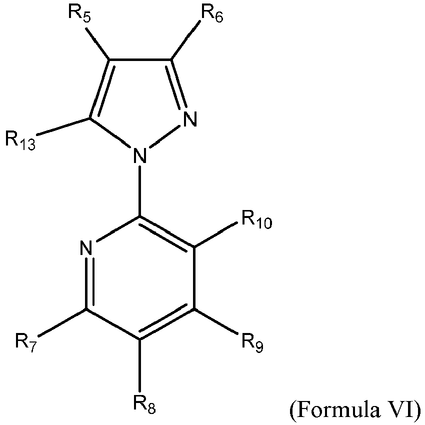
   each of R₅ - R₁₀ is independently selected from hydrogen and halogen; and
   R₁₃ is an organic acid, the method comprising
      I) forming a mixture comprising
         A) a compound of Formula V, wherein 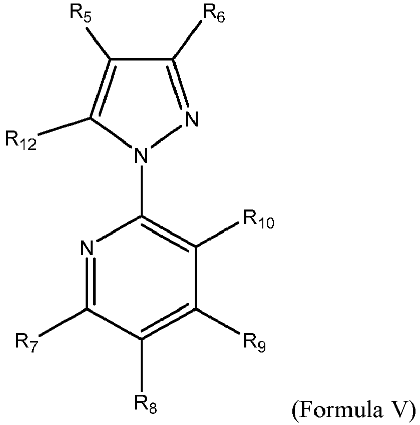
            each of R₅ - R₁₀ is independently selected from hydrogen and halogen;
            R₁₂ is selected from ether, ester, and nitrile; and
            wherein the compound of Formula V is prepared according to a method comprising
               i) forming a mixture comprising
                  a) a compound of Formula III, wherein 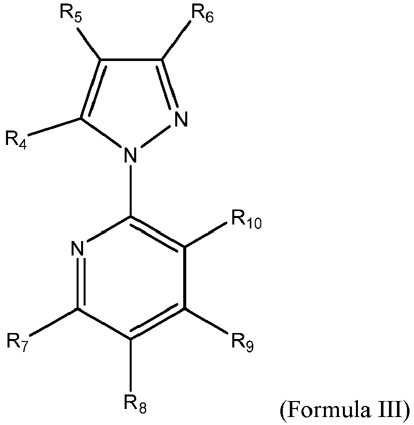
                     R₄ is hydrogen;
                     each of R₅ - R₁₀ is independently selected from hydrogen and halogen; and
                     wherein the compound of Formula III is prepared according to a method comprising
                        IA) forming a mixture comprising
                           AA) a compound of Formula II, wherein 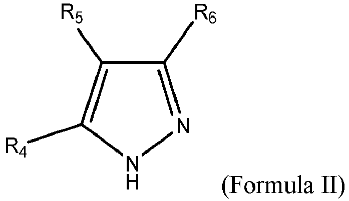
                              each of R₄, R₅, and R₆ is independently selected from hydrogen and halogen;
                              wherein at least one of R₄, R₅, and R₆ is hydrogen; and
                              wherein the compound of Formula II is prepared according to a method comprising
                                 ia) forming a mixture comprising
                                    aa) a compound of Formula VIII, wherein 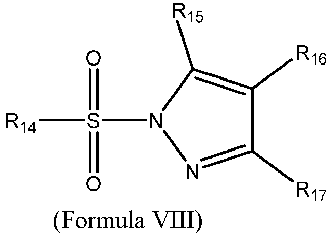
                                       R₁₄ is selected from substituted or unsubstituted aryl, substituted or unsubstituted heteroaryl, substituted or unsubstituted carbocycle, and substituted or unsubstituted heterocycle;
                                       each of R₁₅, R₁₆, and R₁₇ is independently selected from hydrogen and halogen; and
                                       wherein at least one of R₁₅, R₁₆, and
                                       R₁₇ is a halogen;
                                    bb) a solvent; and
                                    cc) an inorganic base; and
                                 iia) reacting the mixture;
                           BB) a compound of Formula IV, wherein 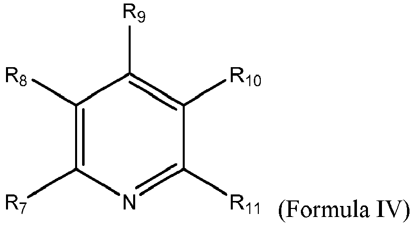 each of R₇ - R₁₁ is independently selected from hydrogen and halogen;
                           CC) a solvent;
                           DD) an inorganic base; and
                           EE) optionally an additive; and
                        IIA) reacting the mixture;
                  b) a solvent;
                  c) an organic compound;
                  d) a compound comprising a metal; and
                  e) optionally an additive; and
               ii) reacting the mixture; and
         B) a metal hydroxide; and
      II) reacting the mixture.
A2. The method of Aspect A1, wherein the metal hydroxide is selected from alkali hydroxide, alkaline earth metal hydroxide, and combinations thereof.
A3. The method of Aspect A2, wherein the alkali hydroxide is selected from lithium hydroxide, sodium hydroxide, and potassium hydroxide.
A4. The method of Aspect A2, wherein the alkaline earth metal hydroxide is selected from calcium hydroxide and barium hydroxide.
A5. The method of Aspect A1, wherein the method step II) of reacting the mixture occurs at a reaction temperature in the range of about 0 °C to about 90 °C.
A6. The method of Aspect A1, wherein the compound comprising a metal is selected from a Grignard reagent and a lithium-containing compound.
A7. The method of Aspect A6, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
A8. The method of Aspect A7, wherein the Grignard reagent is *i*Pr₂NMgCl.
A9. The method of Aspect A6, wherein the lithium-containing compound is selected from LDA, nBuLi, and combinations thereof.
A10. The method of Aspect A1, wherein the solvent b) is selected from THF, toluene, 1,4-dioxane, Me-THF, and combinations thereof.
A11. The method of Aspect A10, wherein the solvent b) is THF.
A12. The method of Aspect A1, wherein the organic compound is selected from dimethyl carbonate, N,N-dimethyacetamide, and combinations thereof.
A13. The method of Aspect A12, wherein the organic compound is dimethyl carbonate.
A14. The method of Aspect A1, wherein the method step ii) of reacting the mixture occurs at a reaction temperature in the range of about 0 °C to about 60 °C.
A15. The method of Aspect A14, wherein the method step ii) of reacting the mixture occurs at a reaction temperature in the range of about 0 °C to about 30 °C.
A16. The method of Aspect A1, wherein R₅ and R₆ of Formula III are each independently hydrogen.
A17. The method of Aspect A1, wherein the inorganic base DD) is selected from powder sodium hydroxide, powder potassium hydroxide, potassium carbonate, potassium phosphate, powder sodium methoxide, powder potassium *t*-butoxide, and combinations thereof.
A18. A method of preparing a compound of Formula VI, wherein 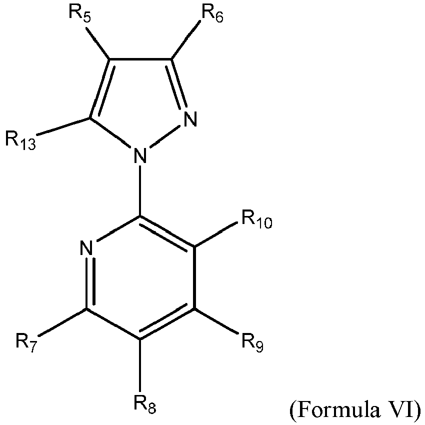
   each of R₅ - R₁₀ is independently selected from hydrogen and halogen; and
   R₁₃ is an organic acid, the method comprising
      I) forming a mixture comprising
         A) a compound of Formula III, wherein 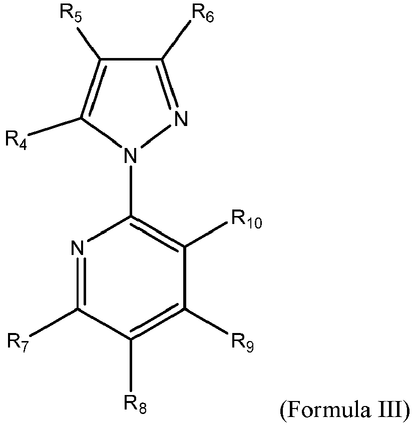
            R₄ is hydrogen;
            each of R₅ - R₁₀ is independently selected from hydrogen and halogen; and
            wherein the compound of Formula III is prepared according to a method comprising
               i) forming a mixture comprising
                  a) a compound of Formula II, wherein 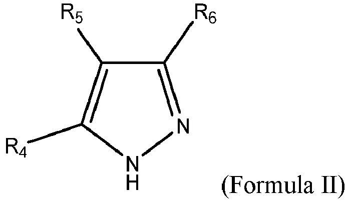
                     each of R₄, R₅, and R₆ is independently selected from hydrogen and halogen;
                     wherein at least one of R₄, R₅, and R₆ is hydrogen; and
                     wherein the compound of Formula II is prepared according to a method comprising
                        IA) forming a mixture comprising
                           AA) a compound of Formula VIII, wherein 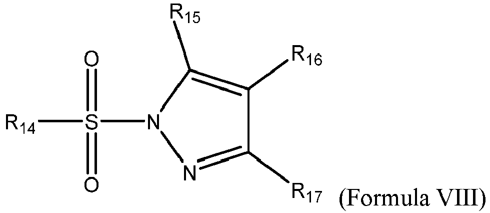
                              R₁₄ is selected from substituted or unsubstituted aryl, substituted or unsubstituted heteroaryl, substituted or unsubstituted carbocycle, and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           BB) a solvent; and
                           CC) an inorganic base; and
                        IIa) reacting the mixture;
                  b) a compound of Formula IV, wherein 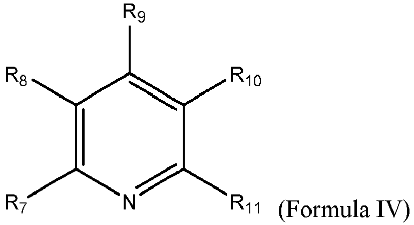 each of R₇ - R₁₁ is independently selected from hydrogen and halogen;
                  c) a solvent;
                  d) an inorganic base; and
                  e) optionally an additive; and
               ii) reacting the mixture;
         B) a carbonyl-containing compound;
         C) a solvent;
         D) a compound comprising a metal; and
         E) optionally an additive; and
      II) reacting the mixture.
A19. A method of preparing a compound of Formula II, wherein 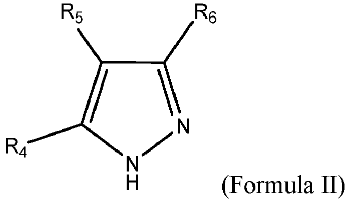
   each of R₄, R₅, and R₆ is independently selected from hydrogen and halogen; and
   wherein at least one of R₄, R₅, and R₆ is hydrogen, the method comprising
      I) forming a mixture comprising
         A) a compound of Formula VIII, wherein 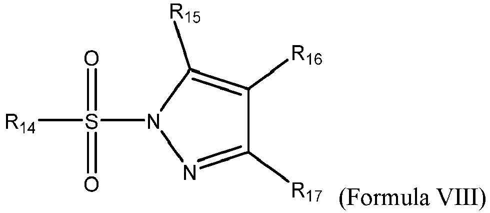
            R₁₄ is selected from substituted or unsubstituted aryl, substituted or unsubstituted heteroaryl, substituted or unsubstituted carbocycle, and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
         B) a solvent; and
         C) an inorganic base; and
      II) reacting the mixture.
A20. The method of Aspect A19, wherein the solvent is selected from THF, toluene, heptanes, Me-THF, and combinations thereof.

## Claims

1. A method of preparing a compound of Formula VI, wherein 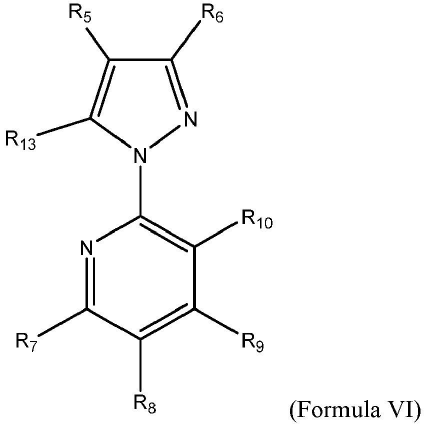
each of R₅ - R₁₀ is independently selected from hydrogen and halogen; and
R₁₃ is an organic acid, the method comprising
I) forming a mixture comprising
A) a compound of Formula V, wherein 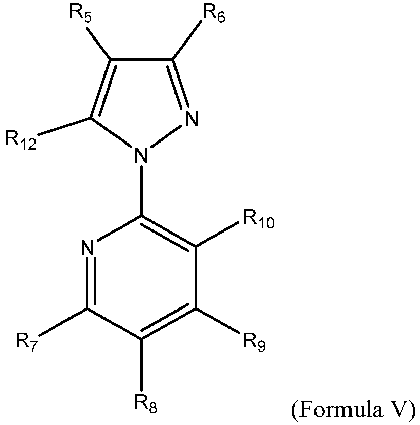
each of R₅ - R₁₀ is independently selected from hydrogen and halogen;
R₁₂ is selected from ether, ester, and nitrile; and
wherein the compound of Formula V is prepared according to a method comprising
i) forming a mixture comprising
a) a compound of Formula III, wherein 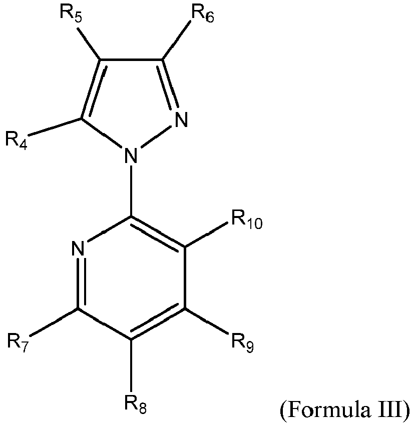
R₄ is hydrogen;
each of R₅ - R₁₀ is independently selected from hydrogen and halogen; and
wherein the compound of Formula III is prepared according to a method comprising
IA) forming a mixture comprising
AA) a compound of Formula II, wherein 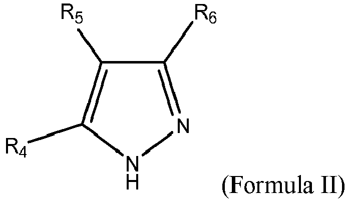
each of R₄, R₅, and R₆ is independently selected from hydrogen and halogen;
wherein at least one of R₄, R₅, and R₆ is hydrogen; and
wherein the compound of Formula II is prepared according to a method comprising
ia) forming a mixture comprising
aa) a compound of Formula VIII, wherein 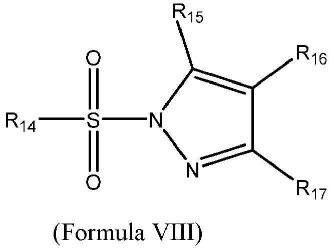
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
bb) a solvent; and
cc) an inorganic base; and
iia) reacting the mixture;
BB) a compound of Formula IV, wherein 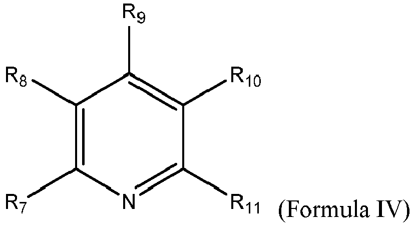 each of R₇ - R₁₁ is independently selected from hydrogen and halogen;
CC) a solvent;
DD) an inorganic base; and
EE) optionally an additive; and
IIA) reacting the mixture;
b) a solvent;
c) an organic compound;
d) a compound comprising a metal; and
e) optionally an additive; and
ii) reacting the mixture; and
B) a metal hydroxide; and
II) reacting the mixture.

2. The method of claim 1, wherein the metal hydroxide is selected from alkali hydroxide, alkaline earth metal hydroxide, and combinations thereof, preferably
(A) wherein the alkali hydroxide is selected from lithium hydroxide, sodium hydroxide, and potassium hydroxide, or
(B) wherein the alkaline earth metal hydroxide is selected from calcium hydroxide and barium hydroxide.

3. The method of claim 1, wherein the method step II) of reacting the mixture occurs at a reaction temperature in the range of about 0 °C to about 90 °C.

4. The method of claim 1, wherein the solvent b) is selected from THF, toluene, 1,4-dioxane, Me-THF, and combinations thereof, preferably wherein the solvent b) is THF.

5. The method of claim 1, wherein the organic compound is selected from dimethyl carbonate, N,N-dimethyacetamide, and combinations thereof.

6. The method of claim 5, wherein the organic compound is dimethyl carbonate.

7. The method of claim 1, wherein the method step ii) of reacting the mixture occurs at a reaction temperature in the range of about 0 °C to about 60 °C.

8. The method of claim 7, wherein the method step ii) of reacting the mixture occurs at a reaction temperature in the range of about 0 °C to about 30 °C.

9. The method of claim 1, wherein R₅ and R₆ of Formula III are each independently hydrogen.

10. The method of claim 1, wherein the inorganic base DD) is selected from powder sodium hydroxide, powder potassium hydroxide, potassium carbonate, potassium phosphate, powder sodium methoxide, powder potassium t-butoxide, and combinations thereof.

11. A method of preparing a compound of Formula VI, wherein 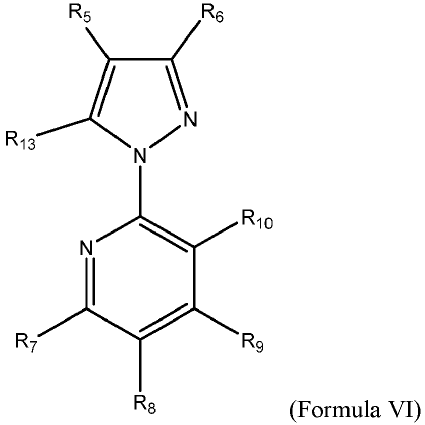
each of R₅ - R₁₀ is independently selected from hydrogen and halogen; and
R₁₃ is an organic acid, the method comprising
I) forming a mixture comprising
A) a compound of Formula III, wherein 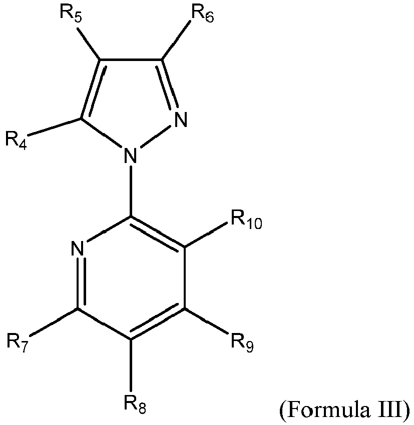
R₄ is hydrogen;
each of R₅ - R₁₀ is independently selected from hydrogen and halogen; and
wherein the compound of Formula III is prepared according to a method comprising
i) forming a mixture comprising
a) a compound of Formula II, wherein 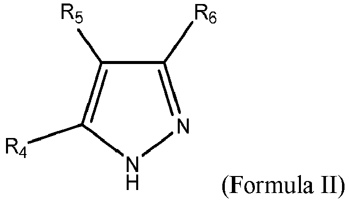
each of R₄, R₅, and R₆ is independently selected from hydrogen and halogen;
wherein at least one of R₄, R₅, and R₆ is hydrogen; and
wherein the compound of Formula II is prepared according to a method comprising
IA) forming a mixture comprising
AA) a compound of Formula VIII, wherein 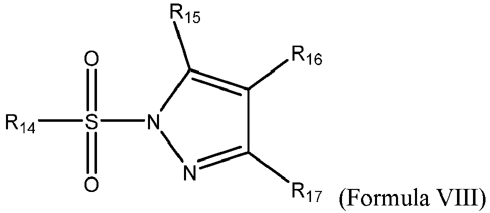
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
BB) a solvent; and
CC) an inorganic base; and
IIa) reacting the mixture;
b) a compound of Formula IV, wherein 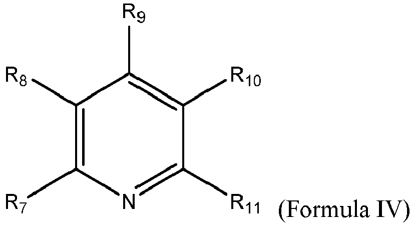 each of R₇ - R₁₁ is independently selected from hydrogen and halogen;
c) a solvent;
d) an inorganic base; and
e) optionally an additive; and
ii) reacting the mixture;
B) a carbonyl-containing compound;
C) a solvent;
D) a compound comprising a metal; and
E) optionally an additive; and
II) reacting the mixture.

12. The method of claim 1 or claim 11, wherein the compound comprising a metal is selected from a Grignard reagent and a lithium-containing compound.

13. The method of claim 12, wherein:
(A) the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl_{•}LiCl, *i*Pr₂NMgBr•LiCl, and combinations thereof, preferably wherein the Grignard reagent is *i*Pr₂NMgCl; or
(B) the lithium-containing compound is selected from LDA, nBuLi, and combinations thereof.

14. A method of preparing a compound of Formula II, wherein 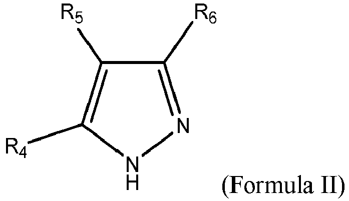
each of R₄, R₅, and R₆ is independently selected from hydrogen and halogen; and
wherein at least one of R₄, R₅, and R₆ is hydrogen, the method comprising
I) forming a mixture comprising
A) a compound of Formula VIII, wherein 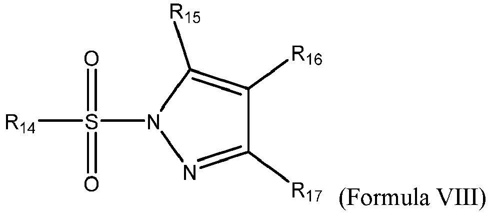
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
B) a solvent; and
C) an inorganic base; and
II) reacting the mixture.

15. The method of claim 14, wherein the solvent is selected from THF, toluene, heptanes, Me-THF, and combinations thereof.
